# EUROPEAN PATENT APPLICATION

(11) **EP 4 046 652 A1**
(43) Date of publication of application: **24.08.2022**
(21) Application number: 21158777.9
(22) Date of filing: 23.02.2021
(51) Int. Cl.: A61K 39/02, G01N 33/558, G01N 33/569, G01N 33/68, A61P 31/04, C07K 14/195

(54) **COMPOSITIONS AND METHODS FOR DIAGNOSING, TREATING AND PREVENTING CARRION'S DISEASE**

(71) Applicant: Johann Wolfgang Goethe-Universität Frankfurt, 60323 Frankfurt am Main (DE); Universidad Peruana Cayetano Heredia, Lima (PE)
(72) Inventor: KEMPF, Volkhard, 63303 Dreieich (DE); DICHTER, Alexander, 60486 Frankfurt am Main (DE); WENIGMANN, Anne, 61352 Bad Homburg (DE)
(74) Representative: Kuttenkeuler, David

(57) **Abstract**

The invention is based on the identification of immunodominant proteins of *Bartonella bacilliformis.* The immunodominant proteins identified in context of the invention are used as protein and mRNA vaccines as well as in the development of immunological detection assays for the diagnosis of an infection with *Bartonella bacilliformis.*

SEQUENCE LISTING

<110>  Johann Wolfgang Goethe-Universität Frankfurt am Main
       Universidad Peruana Cayetano Heredia

<120>  COMPOSITIONS AND METHODS FOR DIAGNOSING, TREATING AND PREVENTING
       CARRION'S DISEASE

<130>  U31020EP

<160>  14

<170>  PatentIn version 3.5

<210>  1
<211>  551
<212>  PRT
<213>  B. bacilliformis

<400>  1

Met Lys Glu Ile Gln Pro Gln Arg Gly Ala Val Gly Cys Val Leu Val
1               5                   10                  15

Ser Tyr Phe Trp Leu Lys Arg Phe Leu Gly Ile Ala Ile Ala Ala Leu
            20                  25                  30

Leu Ser Thr Ile Leu Pro Ala Glu Gly Gln Pro Ile Glu His Val Thr
        35                  40                  45

Leu Ser Glu His Asn Gly Val Pro Ile Val Asp Gly Ala Phe Glu Leu
    50                  55                  60

Gln Gln Ser Ser Asn Ser Gly Asp Arg Asp Cys Arg Asn Met Asn Glu
65                  70                  75                  80

Val Ile Asn Arg Leu Ser Arg Glu Val Gly Asp Gly Arg Val Leu Pro
                85                  90                  95

Leu Glu Leu Glu Glu Lys Tyr Cys Glu Thr Ile Val Glu Arg Leu His
            100                 105                 110

Asn Leu Gly Asp Ser Asn Gly Ile Ser Leu Gln Ala Leu Asn Met Ile
        115                 120                 125

Glu Gly Thr Gly Val His Lys Gly Asn Ser Gly Lys Phe Lys Val Tyr
    130                 135                 140

Ser Val Gly Leu Gly His Tyr Ser Gly Val Leu Gly Val Gly Ala Ala
145                 150                 155                 160

Thr Ala Val Asn Ala Phe Gly Ala Gln Ala Tyr Gly Phe Gly Ala Arg
                165                 170                 175

Ala Met Ala Pro Ala Thr Ile Ala Val Gly Ile Gly Ala Asp Ala Gln
        180                 185                 190

Asn Met Gly Ala Val Ala Val Gly Gly Ile Ala Ser Ala Lys Gly Lys
        195                 200                 205

Asn Ser Leu Ala Leu Gly Pro Gln Thr Leu Ala Tyr Ser Asp Gly Ser
    210                 215                 220

Val Ala Ile Gly Gly Asp Pro Asp Ser Glu Phe Gly Ala Arg Ala Leu
225                 230                 235                 240

Ala Thr Asn Ser Leu Ser Met Gly Ala Lys Ser Tyr Thr Gly Glu Asn
                245                 250                 255

Ser Glu Ala Ala Ile Ala Val Gly Leu Gly Ala Gln Val Arg Asp Gly
            260                 265                 270

Ser Pro Gly Gly Val Ala Leu Gly Trp Gly Ala Phe Val Ala Glu Gly
        275                 280                 285

Ala Glu Gly Gly Leu Ala Leu Gly Arg Asn Ser His Val Asp Val Asn
    290                 295                 300

Gln Gly Val Ala Leu Gly Val Gly Ser Arg Val Asn Thr Pro Ala Gly
305                 310                 315                 320

Val Ala Gly Tyr Asp Pro Arg Ser Asn Asp Glu Thr Lys Asn Thr Asn
                325                 330                 335

Ser Ala Trp Lys Ser Arg Tyr Gly Ala Val Ser Val Gly Asp Ala Glu
            340                 345                 350

Arg Asp Ile Thr Arg Gln Ile Thr Gly Val Ala Ala Gly Thr Gln Asn
        355                 360                 365

Thr Asp Ala Val Asn Val Ala Gln Leu Gln Ala Leu Arg Asp Ala Thr
    370                 375                 380

Val Leu Tyr Asn Arg Asp Glu Asp Gly Asn Lys Thr Asn Lys Ile Ala
385                 390                 395                 400

Leu Gln Gly Gly Asp Pro Ser Gln Pro Val Ala Leu Asp Asn Leu Ala
                405                 410                 415

Asp Gly Asn Val Thr Glu Gly Ser Lys Gln Ala Val Asn Gly Gly Gln

            420                 425                 430

Leu Tyr Asp Phe Met Gln Val Gln Thr Asp Tyr Thr Lys Glu Gln Thr
        435                 440                 445

Arg Arg Val Leu Lys Asp Ala Lys Gly Tyr Thr Asp Lys Lys Phe Asn
    450                 455                 460

Ala Leu Ser Tyr Glu Ile Asp Gly Ala Arg Lys Glu Ala Arg Gln Gly
465                 470                 475                 480

Ala Ala Ile Gly Leu Ala Val Ser Asn Leu Arg Tyr Asp Asp Thr Pro
                485                 490                 495

Gly Ser Leu Ser Leu Ala Phe Gly Thr Gly Met Trp Arg His Gln Tyr
            500                 505                 510

Gly Phe Ala Val Gly Ala Gly Phe Met Ser Val Asp Gly Lys Val Ser
        515                 520                 525

Ala Asn Val Ser Ala Ala Thr Ala Gly Gly His Trp Gly Val Gly Ala
    530                 535                 540

Gly Val Ser Val Thr Leu Asn
545                 550

<210> 2
<211> 1235
<212> PRT
<213> B. bacilliformis

<400> 2

Met Lys Lys Asn Ser Thr Thr Lys Gly Asn Asn Leu Asp Ala Lys Gly
1               5                   10                  15

Asn Asp Leu Asp Asp Cys His Phe Pro Tyr Arg Ser Phe Leu Phe Lys
            20                  25                  30

Ala Val Ile Leu Gly Thr Ala Met Thr Ala Leu Leu Ser Ser Val Ala
        35                  40                  45

Leu Val Phe Pro Val His Ser Ala Ser Ile Glu Glu Thr Ala Gln Asn
    50                  55                  60

Ala Lys Lys Ser Glu Ser Leu Lys Asp Tyr Arg Ser Gln Asn Lys Lys
65                  70                  75                  80

Gly Ala Asp Thr Ser His Leu Trp Gly Leu Asn Lys Asn Ile Ala Leu

85                  90                  95

Met Asp Gly Val Ala Gln Ala Lys His Lys Thr Leu Asn Val Ala Ala
            100                 105                 110

Pro Thr Arg Asn Leu Leu Leu Lys Glu Asn Ile Thr Ile Gly Ser Leu
        115                 120                 125

Met Asn Ala Ala Asp Arg Met Ala Thr Asp Ser Ile Ser Ile Met His
    130                 135                 140

Ser Asn Tyr Glu Gly Arg Asp Ala Pro Ile Asn Cys Ala Ile Ser Phe
145                 150                 155                 160

Asp Thr Pro Thr Ala Met Cys Phe Gly Phe Arg Glu Arg Met Asp Ser
                165                 170                 175

Gln Asp Leu Pro Ser Ile Ser Leu Ala His Tyr Ala His Ser Tyr Asp
            180                 185                 190

Asn Arg Val Ser Asp Asn Tyr Gly Ser Gln Ile Asp Lys Ala Ser Lys
        195                 200                 205

Glu Gly Asp Val Gln Ser Asp Ser Ser Arg Ser Leu Ser Lys Glu Ala
    210                 215                 220

Glu Gly Glu Glu Lys Thr Tyr Leu Glu Glu Gly Ala Lys Leu Ala Asp
225                 230                 235                 240

Ile Leu Asn Asp Thr Pro Ile Val Thr Thr Phe Met Thr Gln Asp Lys
                245                 250                 255

Pro Asn Leu Gly Gly Gly Asp Val Phe Ile Ser Ala Asn Asn Ser Ser
            260                 265                 270

Gly Asp Lys Ile Tyr Ser Arg Phe Gly Ile Ala Glu Thr Arg Ser Leu
        275                 280                 285

Asp Asp Leu Leu Cys Asn Thr Ala Ser Lys Asp Ile Leu Cys Ile Gly
    290                 295                 300

Gln Leu Thr Ala Ala Lys Gly Phe Ala Ser Val Ala Leu Gly Tyr Gly
305                 310                 315                 320

Ser Gln Ala Ile Arg Gly Pro Asn Val Ala Gly Tyr Asp Pro Ile Thr
                325                 330                 335

Asp Gly Ile Ser Thr Ser Ser Asp Tyr Leu Trp Lys Ser Thr His Gly
        340                 345                 350

Glu Leu Gly Ile Gly Asn Met Asp Ile Lys Arg Ser Arg Gln Ile Thr
    355                 360                 365

Gly Val Ala Ala Gly Arg Glu Asp Asn Glu Ala Val Asn Val Ala Gln
370                 375                 380

Leu Lys Ala Leu Arg Lys Trp Val Gln Thr Arg Asp Thr Asp Ile Ser
385                 390                 395                 400

Asp Ile Ile Gln Phe Arg Leu Leu Asn Val Tyr Gly Ser Gly Gln Ile
                405                 410                 415

Ser Ile Gly Trp Asp Lys Ala Ser Val Gln Ser Ile Ser Val Asn His
            420                 425                 430

Lys Asn Gly Asn Lys Arg Tyr Ile Glu Gly Ile Arg Asp Gly Val Ile
        435                 440                 445

Ala Ser Asn Ser Ser Met Ala Val Thr Gly Gly Gln Leu Tyr Ser Ala
    450                 455                 460

Tyr Lys Thr Val Glu Lys Phe Leu Gly Gly Gly Ala Ser Phe Gly Val
465                 470                 475                 480

Gly Gly Phe Gly Asp Lys Gln Pro Ile Phe Asn Ile Gln Gly Asn Arg
                485                 490                 495

Tyr Arg Asp Val Gly Ala Ala Phe Asp Gly Val Asp Asn Ser Phe Ser
            500                 505                 510

Lys Phe Asp Lys Ser Ile Glu Glu Ile Lys Asn Asp Ile Ser Ser Ala
        515                 520                 525

Met Gly Asp Ser Leu Val Lys Gln Asp Val Lys Thr Gly Ile Ile Thr
    530                 535                 540

Ile Gly Ser Glu Lys Glu Gly Thr Val Ile Asp Ile Ala Asn Ser Lys
545                 550                 555                 560

Gln Glu Thr Arg Gln Leu Thr Gly Leu Lys Ala Gly Ile Ile Ser Gln
                565                 570                 575

Asn Ser Thr Asp Ala Val Thr Gly Gly Gln Leu Trp Thr Thr Arg Glu
            580                 585                 590

Lys Val Asn Ala Leu Asp Thr Asn Ile Lys Val Ala Ile Leu Asp Ile
        595                 600                 605

Ala Ile Val Lys Asn Asp Val Ser Ile Leu Glu Thr Asn Val Asn Asn
    610                 615                 620

Ala Ala Ala Asn Ile Ser Gln Tyr Leu Gly Ser Tyr Ala Asp Val Leu
625                 630                 635                 640

Lys Gly Val Ala Pro Ile Tyr Glu Ile Gln Gly Gln Thr Tyr Arg Asp
                645                 650                 655

Val Gly Ala Ala Phe Ala Gly Val Asp Asp Ser Phe Gly Lys Phe Asp
            660                 665                 670

Lys Ser Ile Glu Glu Ile Lys Asn Asp Ile Ser Ser Ala Met Gly Asp
        675                 680                 685

Ser Leu Val Lys Gln Asp Val Lys Thr Gly Ile Ile Thr Ile Gly Ser
    690                 695                 700

Glu Lys Glu Gly Thr Val Ile Asp Ile Ala Asn Ser Lys Gln Glu Thr
705                 710                 715                 720

Arg Gln Leu Thr Gly Leu Lys Ala Gly Ile Ile Ser Gln Asn Ser Thr
                725                 730                 735

Asp Ala Val Thr Gly Gly Gln Leu Trp Thr Thr Arg Glu Lys Val Asn
            740                 745                 750

Ala Leu Asp Thr Asn Ile Lys Ala Ala Val Leu Asp Ile Ala Ile Leu
        755                 760                 765

Glu Gly Asp Val Ser Ile Leu Glu Thr Asn Val Asn Asn Ala Ala Ala
    770                 775                 780

Asn Ile Ser Gln Tyr Leu Gly Gly Tyr Ala Asp Val Leu Lys Gly Val
785                 790                 795                 800

Ala Pro Ile Tyr Glu Ile Gln Gly Gln Thr Tyr Arg Asp Ile Ser Ser
                805                 810                 815

Ala Phe Asp Gly Val Asp Lys Phe Leu Thr Lys Ile Asn Ser Gly Asn
            820                 825                 830

Leu Glu Gln Asp Ala Leu Leu Trp Ser Asp Glu Glu Asn Ala Phe Val
        835                 840                 845

Ala Leu His Ala Ala Glu Lys Gly Lys Lys Ser Gln Asn Ser Lys Leu
    850                 855                 860

Lys Phe Leu Leu Asp Gly Asp Val Ser Glu Gly Ser Thr Glu Ala Ile
865                 870                 875                 880

Thr Gly Asn Gln Leu Tyr Val Val Ser Asn Gln Leu Ala Leu Tyr Leu
                885                 890                 895

Gly Gly Gly Ala Gly Tyr Asn Asn Gly Gln Trp Ile Ala Pro Thr Phe
            900                 905                 910

Lys Val Ala Gln Phe Ser Asn Asn Gly Ser Tyr Ser Ala Glu Lys Thr
        915                 920                 925

Tyr Asn Asn Val Ala Asp Ala Phe Asp Gly Val Asn Lys Ser Met Ala
    930                 935                 940

Asp Ile Asn Asn Arg Val Asn Glu Ala Thr Gln Asn Gly Gly Lys Asp
945                 950                 955                 960

Ser Leu Asn Trp Asn Glu Asn Ile Gly Thr Phe Asp Ala Ser His Asn
                965                 970                 975

Gly Lys Pro Gly Ala Val Thr Asn Val Ala Asp Gly Lys Val Glu Gln
            980                 985                 990

Gly Ser Lys Asp Ala Ile Asn Gly  Gly Gln Leu Trp Asp  Val Lys Glu
        995                 1000                1005

Asp Val  Lys Gly Val Gln Asn  Gln Val Asn Asp Leu  Lys Asp Lys
    1010                1015                1020

Val Asn  Gly Phe Asp Asp Lys  Leu Asn Gly Ile Glu  Asn Ala Val
    1025                1030                1035

Asp Asp  Met Lys Asp Lys Ile  Lys Asp Ala Thr Asp  Gly Val Val
    1040                1045                1050

Ser Tyr  Asp Lys Asp Lys Asn  Gly Lys Lys Thr Asn  Lys Ile Thr
    1055                1060                1065

Leu Gln  Gly Gly Asp Glu Ser  Lys Pro Val Thr Leu  Asp Asn Val
    1070                1075                1080

Ala Asp  Gly Lys Val Glu Lys  Gly Ser Gln Glu Ala  Val Asn Gly

1085                    1090                    1095

Gly Gln  Leu His Asp Tyr Thr  Lys Glu Lys Met Gln  Leu Val Leu
    1100                    1105                    1110

Asp Asp  Ala Lys Lys Tyr Thr  Asp Glu Lys Ile Lys  Asn Ile Val
    1115                    1120                    1125

Val Asp  Phe Val Asp Glu Ala  Val Ala Lys Ala Lys  Asp Tyr Thr
    1130                    1135                    1140

Asp Gln  Lys Phe Asn Val Leu  Ser Tyr Glu Ile Asp  Gly Ala Arg
    1145                    1150                    1155

Lys Glu  Ala Arg Gln Gly Ala  Ala Ile Gly Leu Ala  Val Ser Asn
    1160                    1165                    1170

Leu Arg  Tyr Asp Asp Thr Pro  Gly Lys Phe Ser Leu  Ala Phe Gly
    1175                    1180                    1185

Ala Gly  Met Trp Arg Gly Glu  Ser Ala Val Ala Leu  Gly Ser Ala
    1190                    1195                    1200

Tyr Thr  Ser Glu Asn Gly Arg  Val Arg Ser Asn Leu  Ser Ala Thr
    1205                    1210                    1215

Ser Gly  Gly Gly His Trp Gly  Val Gly Ala Gly Leu  Ser Leu Thr
    1220                    1225                    1230

Leu Asn
    1235

<210>  3
<211>  124
<212>  PRT
<213>  B. bacilliformis

<400>  3

Met Lys His Ile Tyr Ser Ile Ser Cys Leu Thr Ile Thr Ala Leu Leu
1               5                   10                  15

Leu Ala Ala Cys Ser Phe Ser Gln Tyr Tyr Gly Gln Tyr Pro Phe Val
            20                  25                  30

Arg Asn Ile Pro Asn Gly Ile Asp Gly Glu Trp Ile Asp Thr Asn Gly
        35                  40                  45

Ile Val Ser Ser Phe His Asn Gly Ile Phe Glu Thr Arg Ala Ala Asp

50                  55                  60

Thr Glu Glu Lys Leu Ser Glu Gly Ile Tyr Asn Tyr Val Asn Thr Gln
65                  70                  75                  80

His Val Glu Ile Glu Ile His Ser Ile Leu Arg Gly Thr Ile Ser Arg
                85                  90                  95

Ala Asp Cys Thr Ile Ser Asn Asn Ala Met Gln Leu Phe Cys Thr Ser
            100                 105                 110

Ser Asn Gly Leu Gln Phe Ser Leu Asn Arg Lys Ile
        115                 120

<210>  4
<211>  406
<212>  PRT
<213>  B. bacilliformis

<400>  4

Met Asn Ile Lys Thr Leu Leu Leu Cys Ser Thr Val Ala Phe Ile Gly
1               5                   10                  15

Ile Ser Gly Ala His Ala Ala Pro Asp Ile Val Glu Lys Pro Lys Ser
            20                  25                  30

Ile Lys Tyr Leu Arg Ala Cys Asp Ala Tyr Gly Ala Gly Tyr Phe Tyr
        35                  40                  45

Ile Pro Gly Thr Glu Thr Cys Met Lys Leu Ser Gly Tyr Val Arg Ala
    50                  55                  60

Ser Phe Lys Gly Gly His Asp Val Asp Ala Lys Thr Asn Ala Asp Leu
65                  70                  75                  80

Asp Gln His Lys Lys Thr Tyr Asp Val Ser Ser Arg Leu Ala Leu Ile
                85                  90                  95

Phe Gln Ala Ala Ser Glu Thr Glu Leu Gly Thr Leu Arg Ser Tyr Ala
            100                 105                 110

Arg Leu Thr Ser Asp Trp Val Asn Gly Lys Ser Gly Ala Gly Gly Arg
        115                 120                 125

Leu Asn Ala Ala Tyr Ile Arg Leu Gly Gly Phe Ser Val Gly Leu Asp
    130                 135                 140

Asp Thr Ile Phe Ser Thr Trp Thr Ser Tyr Gly Glu Val Ile Asn Asp

145                 150                 155                 160

Asp Leu Ile Ala Pro Ala Gly Asn Val Arg Thr Asn Phe Val Ser Tyr
                165                 170                 175

Thr Phe Ala Ser Asn Thr Gly Phe Ala Ala Ile Ile Gly Ala Glu Leu
            180                 185                 190

Gly Asn Ala Ser Asp Pro Ile Gly Pro Asp Lys Lys Tyr Tyr Tyr Ile
        195                 200                 205

Asp Lys Ser Asp Gln Val Ser Ile Val Ala Asp Asn Asp Leu Leu Ser
    210                 215                 220

Lys Gln Thr Lys Asp Tyr Ile Pro His Ile Leu Leu Gly Ala Gln Phe
225                 230                 235                 240

Val Gln Gly Trp Gly Gly Val Ser Thr Ile Thr Val Tyr Asp Ser His
                245                 250                 255

Tyr Lys Lys Trp Ala Asn Lys Thr Arg Leu Asp Phe Lys Val Asn Asp
            260                 265                 270

Asp Ile Asn Val Trp Val Thr Gly Gly Tyr Lys Thr Asn Val Asp Tyr
        275                 280                 285

Tyr Thr Ala Asp Glu Gln Asn Val Leu Ser Arg Glu Asn Thr Thr Ile
    290                 295                 300

Tyr Ala Gly Trp Gly Gly Lys Trp Ala Ala Trp Thr Gly Val Thr Tyr
305                 310                 315                 320

Gln Ile Thr Pro Glu Ala Lys Leu Asn Thr Gln Ile Ser Tyr Ser Ala
                325                 330                 335

Val Lys Thr Phe Ser Ala Ser Ala Asn Ile Val Tyr Thr Leu Val Pro
            340                 345                 350

Gly Phe Thr Ile Thr Pro Glu Val Ser Tyr Ile Ala Trp Lys Asp Asp
        355                 360                 365

Arg Thr Phe Lys Gly Gln Gln Asp Ser Ala Gln Arg Asn Ser Thr Pro
    370                 375                 380

Asn Tyr Thr His Ala Leu Asn Gly Lys Asn Ala Leu Gln Thr Met Ile
385                 390                 395                 400

Arg Leu Gln Arg Ser Phe
                405

<210>  5
<211>  731
<212>  PRT
<213>  B. bacilliformis

<400>  5

Met Gln Ile Lys Trp Lys Asn Ile Tyr Lys Ser Cys Val Ala Leu Ser
1               5                   10                  15

Ala Leu Ser Ile Trp Ala Pro Ser Phe Val Phe Ala Gln Asn Asn Asp
            20                  25                  30

Glu Gly Ser Val Thr Asp Leu Lys Ser Ile Val Ile Glu Gly Lys Gln
        35                  40                  45

Asp Leu Asp Pro Ser Ser Thr Val Thr Ile Leu Thr Asp Arg Gln Thr
    50                  55                  60

Ala Gln Asn Ile Glu Gln Lys Gln Val Ser Asp Val His Asp Val Asn
65                  70                  75                  80

Arg Leu Asn Ser Ser Ile Ser Tyr Asn Ser Ser Asn Asp Ser Phe Ile
                85                  90                  95

Val Arg Gly Leu Asp Ala Asn Arg Val Leu Thr Thr Ile Asp Gly Ile
            100                 105                 110

Pro Leu Pro Trp Leu Asn Asp Glu Ala Arg Ser Val Lys Gly Gly Asn
        115                 120                 125

Ser Thr Phe Asp Phe Asn Thr Leu Ser Thr Phe Asp Leu Ile Gln Gly
    130                 135                 140

Ser Asp Ser Ser Ile Tyr Gly Ser Gly Ala Leu Gly Gly Ile Ile Ala
145                 150                 155                 160

Leu Arg Thr Leu Asn Pro Glu Asp Leu Leu Thr Glu Glu Lys Asn Trp
                165                 170                 175

Gly Ser Leu Thr Lys Gly Ser Tyr Asn Ser Val Asp Asn Ser Trp Gln
            180                 185                 190

Val Asp Gln Ala Tyr Ala Val Arg Ala Asn Gln Thr Phe Val Leu Phe
        195                 200                 205

Gln Gly Ser Leu Val Gly Gly His Glu Arg Lys Asn Met Gly Ser Ile
    210                 215                 220

Gly Asp Tyr Gly Thr Lys Arg Thr Leu Ala Asn Pro Ala Asp Phe Asp
225                 230                 235                 240

Gln Asn Asn Leu Leu Phe Lys Val His Gln Tyr Phe Asp His Asn His
                245                 250                 255

Arg Leu Ser Phe Thr Ala Glu Arg Phe His Tyr Asn Lys Asp Thr His
            260                 265                 270

Val Met Asn Ala Pro Ala Ile Tyr Phe Pro Glu Ser Val His Glu Glu
        275                 280                 285

Asn Lys Lys Ser Arg Glu Arg Phe Ser Leu Ser Tyr Asp Tyr Glu Gly
    290                 295                 300

Ser Gln Asp Thr Leu Leu Asp Ala Phe His Gly Gln Ile Tyr Trp Gln
305                 310                 315                 320

Asn Gln Leu Asn Asn His Ile Leu Ser Gly Asn Arg Val Val Ser Pro
                325                 330                 335

Lys Gly Asn Tyr Leu Arg Asp Asn Gln Ile Arg Asn Thr Thr Tyr Gly
            340                 345                 350

Leu Asn Thr Asn Ser Ile Lys Lys Val Asp Ile Gly Thr Thr Thr Asn
        355                 360                 365

Thr Leu Gln Leu Ala Ile Asn Ile Phe Glu Ser Lys Phe His Gln Tyr
    370                 375                 380

Ala Ser Gly Lys Asp Asn Cys His Leu Pro Glu Asn Ala Arg Gly Cys
385                 390                 395                 400

Ala Phe Leu His Thr Asn Gln Ser Asp Ala Pro Asp Thr Asn Ser Arg
                405                 410                 415

Gly Phe Gly Leu Ala Phe Glu Asn Glu Ile Asp Phe Tyr Arg Tyr His
            420                 425                 430

Val Arg Ile Thr Pro Gly Ile Arg Tyr Asp Trp Tyr Thr His Asn Pro
        435                 440                 445

Gln Glu Thr Ala Ser Tyr Gly Lys Val Pro Ile Ser Gln Lys Leu Pro
    450                 455                 460

Ser Lys Ser Ser Ser Ser His Phe Ser Pro Lys Leu Arg Leu Glu Trp
465                 470                 475                 480

Asp Ile His Asn Gln Val Ala Leu Tyr Ala Gln Trp Ala Gln Ala Phe
                485                 490                 495

Arg Ala Pro Ser Val Ser Glu Leu Tyr Leu Asn Tyr Ile Asn Pro Ser
            500                 505                 510

Phe Tyr Tyr Arg Ala Gly Asn Pro Asp Leu Lys Pro Glu Thr Ser Asn
        515                 520                 525

Gly Tyr Asp Leu Gly Val Lys Tyr Gly Asn Met Asn Phe Gly Gly Ser
    530                 535                 540

Phe Gly Ala Phe Ile Asn Asp Tyr Lys Asn Phe Ile Asp Leu Val Asp
545                 550                 555                 560

Lys Gly Pro Ser Arg Glu Phe Arg Leu Ser Arg Glu His Tyr Val Asn
                565                 570                 575

Arg Ala His Val Arg Ile Phe Gly Val Glu Thr Lys Ala His Trp Asn
            580                 585                 590

Leu Asp Asn Gly Leu His Ser Asp Phe Ser Leu Ser Tyr Ala His Gly
        595                 600                 605

Lys Asp Leu Asp Lys Lys Glu Tyr Leu Ser Ser Ile Pro Ala Leu Lys
    610                 615                 620

Thr Ile Phe Gly Leu Gly Tyr Ala Lys Glu Phe Trp Gly Leu Asp Phe
625                 630                 635                 640

Val Leu Thr Leu Ala Ala Lys Asn Asp Lys Val Ala Lys Lys Ser Ser
                645                 650                 655

Tyr Arg Glu Val Pro Gly Tyr Ser Leu Val Asp Val Leu Ser Trp Trp
            660                 665                 670

Lys Pro Phe Gly Lys Asp Gly Leu Thr Ile Arg Ala Gly Val Tyr Asn
        675                 680                 685

Ile Phe Asn Thr Lys Tyr Trp Asn Ala Ser Asp Leu Pro Ser Val Ser
    690                 695                 700

Pro Arg Gly Glu Ser Pro Leu Pro Lys Asp Tyr Phe Ser Gln Pro Gly
705                 710                 715                 720

Arg Ser Phe Lys Val Ser Phe Val Gln Lys Phe
                725                 730

<210>  6
<211>  397
<212>  PRT
<213>  B. bacilliformis

<400>  6

Met Tyr Leu Lys Thr Leu Asn Asp Ile Phe Arg Tyr Asn Phe Gln Lys
1               5                   10                  15

Ile Ala Leu Leu Ala Ile Ala Thr Ile Ile Thr Gly Cys Ser Ser Gly
            20                  25                  30

Thr Gln Arg Phe Phe Asp Gly Phe Tyr Glu Gly Glu Ala Ser Thr Gln
        35                  40                  45

Leu Thr Glu Asn Thr Ala Met Ser Ala Asp Arg Arg Ile Ile Ser Gln
    50                  55                  60

Ser Ser Val Ile Gln Ser Asp Glu Leu Pro Pro Val Glu Pro Leu His
65                  70                  75                  80

Ser Ser Gly Asp His Asn His Ala Ser Tyr Asn Phe Ser Arg Gln Glu
                85                  90                  95

Ala Gly Ser Ser Phe Arg Glu Asn Arg Val Ser Pro Asp Gly Arg Ile
            100                 105                 110

Met Gly Ser Pro Pro Gln Asn Leu Gly Thr Leu Ser Arg Ser Asn Val
        115                 120                 125

Asn Asn Pro Ser Ile Phe Gln Lys Gly Ser Tyr Ile Val Gln Ser Gly
    130                 135                 140

Asp Thr Leu Phe Ser Ile Ala Gln Gln Lys Gly Ile Ser Val Glu Ser
145                 150                 155                 160

Leu Lys Val Ala Asn Gly Met Gly Asp Asn Ala Ile Tyr Ile Gly Gln
                165                 170                 175

Lys Leu Val Ile Pro Asp Lys Gln Thr Val Val Thr Ser Asn Ala Gln
            180                 185                 190

Asn Asn Ala Val Gly Ser Ser Thr Thr Thr Ser Ser Thr Gln Ser Lys
        195                 200                 205

Gly Met Ser Phe Val Lys Lys Glu Glu Ile Ser Ala Ala Ser Arg Ser
    210                 215                 220

Val Asn Thr Ala Pro Ala Ala Ile Ile Asn Thr Pro Asp Val Lys Lys
225                 230                 235                 240

Asn Val Ser Lys Gln Met Thr Thr Ser Lys Ser Ala Ser Asn Ser Ser
                245                 250                 255

Lys Thr Val Lys Asp Ser Asp Asn Ile Thr Val Pro Gln Ala Thr Gly
            260                 265                 270

Val Ser Lys Met Arg Trp Pro Val Arg Gly Arg Leu Leu Ser His Phe
        275                 280                 285

Gly Gln Lys Lys Gly Thr Ile Thr Asn Arg Gly Ile Asp Ile Asn Val
    290                 295                 300

Pro Glu Gly Ser Ser Val Lys Ala Ala Glu Asn Gly Val Val Ile Tyr
305                 310                 315                 320

Ala Ser Asp Gly Leu Lys Glu Leu Gly Asn Val Val Met Ile Arg His
                325                 330                 335

Glu Asp Asn Ile Ile Thr Ile Tyr Gly His Asn Ser Lys Leu Thr Val
            340                 345                 350

Ser Arg Gly Gln Arg Val Lys Arg Gly Asp Glu Ile Ala Lys Ser Gly
        355                 360                 365

Val Ser Gly Asp Ala Thr Thr Pro Arg Val Tyr Phe Glu Ile Arg Gln
    370                 375                 380

Asn Ser Val Pro Val Asn Pro Ala Gln Tyr Leu Glu Asn
385                 390                 395

<210>  7
<211>  649
<212>  PRT
<213>  B. bacilliformis

<400>  7

Met Lys Leu Lys Leu Phe Phe Phe Phe Leu Ser Leu Leu Leu Thr Ile
1               5                   10                  15

Gln Thr Ser Ser Ala Ala Pro Val Pro Gly Val Ile Gln Lys Pro Pro
            20                  25                  30

Asp Arg Pro His Asp Lys Ala Ile Lys Ile Arg Val His Thr Gly Gly
        35                  40                  45

Glu Tyr Cys Tyr Thr Pro Thr Phe Thr Glu Gly Met Gly Tyr Ile Tyr
    50                  55                  60

Ile Asn Asp Cys Ser Ser Ser Thr Val Lys Ala Arg Tyr Asp Leu Phe
65                  70                  75                  80

Gln Arg Val Ala Trp Asn Ile Asn Asp Thr Trp Leu Cys Met Thr Ala
                85                  90                  95

Pro Glu Ser Ile Thr Gly Ser Asp Ser Asn Ser Ser Ala Asn Trp Asp
            100                 105                 110

Tyr Leu Thr Leu Lys Pro Cys Val Ile Asn Asp Gln Asn Gln Arg Trp
        115                 120                 125

Ile Ile Lys Asp Gly Gly Phe Tyr Thr Ala Asp Glu Arg Phe Arg Val
    130                 135                 140

Lys Asp Thr His Trp Tyr Ala Tyr Ile Ser Lys Asp Ser Lys Ala Ser
145                 150                 155                 160

Tyr Asp His Gln Leu Thr Phe Glu Met Lys Asp Trp Ser Ser Thr Val
                165                 170                 175

Ser Pro Pro Gly Asn Ile Ser Phe Lys Thr Tyr Val Ala Trp Leu Tyr
            180                 185                 190

Val Ser Val Tyr Pro Pro Leu Phe Ala Leu Tyr Tyr Leu Gln Asn Asp
        195                 200                 205

Ser Ser Ser Ile Ser Thr Thr Pro Leu Pro Leu Tyr Tyr Asn Pro Glu
    210                 215                 220

Asn Gly His Ile Ala Gln Tyr Tyr Asp Ser Tyr Gly Ser Phe Tyr Cys
225                 230                 235                 240

Met Ala Ser Lys Gln Thr Thr Ser Gln Asp Trp Asn Trp Val Gln Trp
                245                 250                 255

Thr Glu Cys Thr Asp Lys Ile Pro Ser Lys Lys Asp Ser Thr Phe Trp
            260                 265                 270

Asp Val Ser Gln Leu Asn Gly Asn Glu Gly Phe Ile Leu Asp Tyr Gln

275                 280                 285

Gly Asn Pro Leu Arg Val Thr Gln Tyr Gly Thr Arg Trp Gly Val Pro
    290                 295                 300

Tyr Thr Ala Lys Thr Ser Tyr Ile Pro Lys Asp Thr Thr Asn Ser Pro
305                 310                 315                 320

Thr Ser Lys Phe Ile Leu Ser Thr Asp Ile Glu Gln Trp Asn Arg Tyr
                325                 330                 335

Thr Asn Gly Asn Leu Gly Glu Thr Leu Pro Phe Cys Pro Ala Pro Gly
            340                 345                 350

Lys Lys Gly Ser Ile Leu Gln Thr Ser Gln Ala Arg Lys Gly Leu Phe
        355                 360                 365

Leu Pro Pro Trp Phe Thr Leu Ser Glu Ala Trp Ile Gln Arg Leu Trp
    370                 375                 380

Gln Ile Ala Arg Ser Thr Val Asp Gly Thr Glu Arg Ile Gly Phe Cys
385                 390                 395                 400

Gly Ile Cys Met Leu Gln Thr Ser Gln Met Leu Ala Glu Leu Gln Glu
                405                 410                 415

Asn Tyr Gln Thr Pro Thr Tyr Gln Asp Gly Ala Gly Tyr Phe Phe Asn
            420                 425                 430

Thr Ala Phe Gly Arg Asp Pro Phe Val Ser Leu Arg Ser Arg Phe Pro
        435                 440                 445

His Ile Ala Glu Arg Leu Gln Met Thr Glu Val Tyr Ser Glu Thr Pro
    450                 455                 460

Phe Thr Pro Thr Glu Asp Arg His Glu Arg Ala Asn Arg Ala Cys Asn
465                 470                 475                 480

Val Met Ile Ser Thr Phe Leu Pro Asp Val Met Trp Arg Ser Ser Ala
                485                 490                 495

Val Gly Arg Ala Ser Ala Glu Ile Ala Arg Thr Leu Ile Gly Leu Phe
            500                 505                 510

Arg Ile Pro Pro Gly Thr Ile Gly Phe Val Glu Val Val Arg Glu Asn
        515                 520                 525

Thr Glu Arg Thr Gly Tyr Val Ala His Ala Gln Pro Phe Ile Arg Thr
    530                 535                 540

Gln Glu Gly Ile Ile Leu Ile Pro Thr Asn Ala Pro Gln Leu Ser Leu
545                 550                 555                 560

Asp Glu Phe Arg Gln Ser Leu Thr Pro Ile Pro Thr Thr Gln Gln Gly
                565                 570                 575

Leu Glu Arg Leu Val Leu Tyr Leu Ser Val Arg Gly Ser Gln Pro Ile
            580                 585                 590

Arg Ser Val Ser Thr Phe Glu Met Val Gln Leu Thr Leu Pro Ser Leu
        595                 600                 605

Asn Val Tyr Met Ser Gln Arg Asp Cys Thr Gly Glu Gly Asp His Arg
    610                 615                 620

Arg Gly Ser Arg Gln Leu Pro Ser Thr Ser Ser Ile Asn Glu Cys Ala
625                 630                 635                 640

Ser Gly Gly Gly Arg Cys Ser Pro Arg
                645

<210>  8
<211>  1259
<212>  PRT
<213>  B. bacilliformis

<400>  8

Met Lys Lys Leu Tyr Leu Ile Pro Glu Glu Asn Asp Leu Asn Cys His
1               5                   10                  15

His Ser Leu Arg Cys Ser Pro Leu Val Lys Ala Val Ser Leu Gly Thr
            20                  25                  30

Val Met Ala Ala Leu Leu Ser Ser Val Ser Pro Val Phe Ala Ala Asn
        35                  40                  45

Val Ala Ile Leu Gly Ser Asn Val Gln Ser Ser Asn Asp Gly Ser Ile
    50                  55                  60

Pro Tyr Ser Asn Gly Ser His Gly Ser Ile Val Leu Ala Gly Asp Asn
65                  70                  75                  80

Asn Trp Cys Gly Val Asp Asn Val Thr Asp Arg Gly Lys His Leu Pro
                85                  90                  95

Gln Ser Ser Arg Ala Val Lys Glu Ile Ser Ala Glu Glu Gln Tyr Glu
            100                 105                 110

Arg Phe Val Asn Asn Gly Glu Phe Phe Asn Arg His Pro Tyr Gly Val
        115                 120                 125

Thr Thr Glu Gln Val Lys Trp Glu Ala Asp Gly Val Thr Ser Ser Asn
    130                 135                 140

Gly Gly Tyr Met Gly Ser Ser Thr Thr Gly Gly His Ser Asn Val Met
145                 150                 155                 160

Pro Thr Ala Phe Gly Val Tyr Ser Phe Ala Thr Gly Cys Gly Ser Tyr
                165                 170                 175

Ala Ser Gly Asn Tyr Ser Thr Ala Phe Gly Ala Gly Ala Thr Thr Asn
            180                 185                 190

Gly Gly Gly Ala Gln Ala Tyr Gly Val Ser Ala Leu Ala Lys Gly Gln
        195                 200                 205

Ala Thr Ile Ala Met Gly Val Gly Ala Glu Ala Trp Asp Thr Ser Ala
    210                 215                 220

Val Ala Val Gly Gly Arg Ala Val Ala Leu Gly Lys Asn Ser Val Ala
225                 230                 235                 240

Leu Gly Thr Lys Ala Lys Ala Ser Gly Asp Tyr Gly Val Ala Leu Gly
                245                 250                 255

Thr Glu Ala Leu Ala Lys Gln Arg Gly Ser Ile Ala Ile Gly Gly Asp
            260                 265                 270

Ile Pro Gln Glu Glu Asn Asp Gln Gly Lys Arg Phe Gly Ala Met Val
        275                 280                 285

Glu Ala Glu Asn Ala Ile Ala Ile Gly Val Arg Ser Tyr Ala Ser Glu
    290                 295                 300

Asp Ser Lys Glu Ala Val Ala Ile Gly Leu Ser Ala Gln Val Arg Asp
305                 310                 315                 320

Glu Ala Gly Ile Ala Leu Gly Arg Gly Ala Val Val Arg Gln Gly Gly
                325                 330                 335

Ser Ala Gly Val Ala Ile Gly Ala Ser Ala His Val Ile Ala Asp Thr
            340                 345                 350

Gly Val Ala Leu Gly Thr Ala Ser Ile Ala Ser Thr Glu His Asn Val
        355                 360                 365

Ser Gly Tyr Asn Pro Ala Thr Gly Ala Thr Thr Lys Ser Ser Asp His
    370                 375                 380

Val Trp Val Ser Thr Arg Gly Ala Val Ser Ile Gly Asp Val Ser Gln
385                 390                 395                 400

Lys Val Thr Arg Gln Ile Thr Gly Val Ala Ala Gly Ser Glu Met Thr
                405                 410                 415

Asp Ala Val Asn Val Ala Gln Leu Lys Ala Leu Arg Glu Thr Ile Asp
            420                 425                 430

Lys Gly Trp Met Leu Thr Val Asn Gly Ala Asp Ala Thr Pro Val Gly
        435                 440                 445

Ala Asp Gly Leu Asp Phe Ser Val Glu Asn Pro Asn Leu Asp Thr Pro
    450                 455                 460

Asn Leu Ser Ile Val Arg Ser Gly Asn Lys Leu Thr Phe Asp Leu Ala
465                 470                 475                 480

Glu His Ile Gln Val Gly Arg Ile Thr Thr Gly Leu Ser Ser Leu Ser
                485                 490                 495

Asn Ala Gly Leu Val Ile Lys Gly Cys Asn Asp Gly Thr Ala Cys Lys
            500                 505                 510

Asn Gly Pro Ser Val Thr Ala Gly Gly Ile Asn Ala Gly Asp Lys Thr
        515                 520                 525

Leu Thr Gly Val Ala Asp Ala Val Leu Asp Asn Asp Ala Val Asn Phe
    530                 535                 540

Ser Gln Leu Lys Ala Leu Lys Glu Thr Val Asp Lys Gly Trp Met Leu
545                 550                 555                 560

Thr Val Asn Gly Ser Asp Ala Thr Pro Val Gly Ala Asp Gly Ala Asp
                565                 570                 575

Phe Ser Val Gly Ser Ser Asn Leu Val Ile Thr Lys Asp Asn Lys Asn
            580                 585                 590

Lys Leu Thr Phe Asp Leu Ala Lys Asp Ile Asn Val Asn Ser Ile Lys
        595                 600                 605

Val Gly Lys Ile Ser Leu Asp Asn Asn Ser Val Met Ile Glu Gly Gly
    610                 615                 620

Pro Gln Ile Ser Ala Asp Gly Ala Asp Phe Ala Ser Lys Asn Ile Arg
625                 630                 635                 640

Gly Val Ala Asp Gly Val Leu Asp Gly Asp Ala Val Asn Phe Ser Gln
                645                 650                 655

Leu Lys Ala Leu Lys Glu Thr Val Asp Lys Gly Trp Met Leu Thr Val
            660                 665                 670

Asn Gly Ala Asp Ala Thr Pro Val Gly Ala Asp Gly Ala Asp Phe Ser
        675                 680                 685

Val Gly Ser Ser Asn Leu Val Ile Thr Lys Asp Asn Lys Asn Lys Leu
    690                 695                 700

Thr Phe Asp Leu Ala Glu His Ile Glu Val Gly Arg Ile Thr Thr Gly
705                 710                 715                 720

Leu Ser Ser Leu Ser Asn Ala Gly Leu Val Ile Lys Gly Cys Asn Asp
                725                 730                 735

Gly Thr Ala Cys Lys Asn Gly Pro Ser Val Thr Ala Gly Gly Ile Asn
            740                 745                 750

Ala Gly Asp Lys Thr Leu Thr Gly Leu Ala Asp Gly Val Leu Asp Gly
        755                 760                 765

Asp Ala Val Asn Val Ala Gln Leu Asn Lys Val Lys Glu Ala Ile Lys
    770                 775                 780

Gly Ser Gly Leu Ile Gln Gln Gly Glu Asn Glu Asp Glu Asn Gly Arg
785                 790                 795                 800

Ile Thr Ile Gly Ala Gly Asn Ser Gly Thr Glu Ile Asn Ile Leu Asn
                805                 810                 815

Lys Asp Gly Val Arg Arg Thr Ile Ser Gly Ile Ser Gly Gly Lys Val
            820                 825                 830

Thr Glu Glu Ser Thr Glu Ala Ile Asn Gly Ala Gln Leu Trp Glu Thr
        835                 840                 845

Asn Glu Glu Val His Asn Leu Asn His Arg Val Asp Glu Thr His Thr

850                 855                 860

Asn Val Ser Gln Tyr Phe Gly Gly Gly Ala Asp Ile Ser Ser Gly Lys
865                 870                 875                 880

Ala Pro Thr Tyr Thr Ile Glu Gly Lys Gln Tyr Asn Asn Val Gly Ser
                885                 890                 895

Ala Phe Ser Ala Val Asn Asp Ser Leu Asp Asn Ile Gln Asn Glu Ile
            900                 905                 910

Ser Arg His Phe Gly Gln Ile Tyr Thr Ile Ser Asp Ile Phe Ser His
        915                 920                 925

Tyr Phe Gly Gly Gly Ala Gly Tyr Asn Asn Gly Lys Trp Thr Ala Pro
    930                 935                 940

Thr Tyr Glu Val Ser His Phe Gly Thr Asn Gly Thr Ser Ser Glu Lys
945                 950                 955                 960

Ala Tyr Asn Asn Val Ala Asp Ala Phe Gly Gly Val Asn Glu Ser Met
                965                 970                 975

Ser Gly Met Asn Asp Arg Leu Gln Glu Val Glu Asn Lys Val Asp Gln
            980                 985                 990

Asn Lys Ala Glu Ser Asp Ser Leu  Gly Trp Ser Lys Asp  Lys Gly Ala
        995                 1000                1005

Tyr Asp  Ala Ser His Asp Gly  Lys Pro Ser Lys Ile  Val Asn Val
    1010                1015                1020

Ala Asp  Gly Gln Val Ala Gln  Gly Ser Glu Asp Ala  Val Asn Gly
    1025                1030                1035

Gly Gln  Leu Trp Glu Thr Asn  Glu Arg Val Thr Asn  Val Glu Asn
    1040                1045                1050

Lys Val  Asn Asp Val Ser Lys  Gln Val Asp Ser Ile  Glu Asn Thr
    1055                1060                1065

Val Asn  His Phe Ala Asp Gly  Ala Val Ser Tyr Gln  Arg Asp Glu
    1070                1075                1080

Ser Gly  Lys Lys Thr Asn Lys  Val Val Leu Gln Gly  Gly Asp Ser
    1085                1090                1095

Gly Lys Pro Val Thr Ile Asp Asn Val Ala Asp Gly Lys Ile Glu
    1100                1105                1110

Lys Gly Ser Gln Glu Ala Val Asn Gly Gly Gln Leu His Asp Tyr
    1115                1120                1125

Thr Gln Glu Gln Met Lys Leu Val Leu Asn Asp Ala Lys Lys Tyr
    1130                1135                1140

Thr Asp Gly Lys Ile Lys Asn Ile Val Val Asn Ala Ile Asp Asp
    1145                1150                1155

Ala Val Ser Gln Ala Lys Gly Tyr Thr Asp Met Lys Phe Glu Ala
    1160                1165                1170

Leu Ser Tyr Gly Ile Glu Gly Ala Arg Lys Glu Ala Arg Gln Ala
    1175                1180                1185

Ala Ala Ile Gly Leu Ala Val Ser Asn Leu Arg Tyr Asn Asp Thr
    1190                1195                1200

Pro Gly Arg Leu Ser Phe Ala Phe Gly Ser Gly Met Trp Arg Ser
    1205                1210                1215

Gln Ser Ala Phe Ala Leu Gly Thr Gly Tyr Thr Ser Glu Asp Gly
    1220                1225                1230

Lys Ile Gln Ser Asn Phe Ser Ala Thr Thr Ala Gly Gly His Trp
    1235                1240                1245

Gly Ile Gly Ala Gly Val Ser Ile Thr Leu Asn
    1250                1255

<210> 9
<211> 610
<212> PRT
<213> B. bacilliformis

<400> 9

Met Tyr Lys Lys Tyr Leu Leu Ser Cys Thr Thr Ala Val Ser Phe Leu
1               5                   10                  15

Leu Leu Gly Phe His Leu Asp Ala Asn Ala Gln Arg Ile Val Ile Asp
            20                  25                  30

Asp Gly Arg Thr Glu Asn Ile Ser Asp Leu Thr Leu Ser Ala Val Ser
        35                  40                  45

Pro Arg Pro Glu His Gly Gly Val Tyr Val Thr Asp Gly Thr Leu Asn
    50                  55                  60

Gly Ser Asp Leu Ile Ile Phe Gly Thr Ala Gly Asn Gly Tyr Gly Ala
65                  70                  75                  80

Thr Ala Asp Gly Asn Asn Ala Ile Thr His Asn Gly Gly Lys Ser Ile
                85                  90                  95

Ile Gln Phe Gln Asp Thr Leu Ile Arg Tyr Val Thr Val Gly Leu Phe
            100                 105                 110

Ala Lys Asp Arg Gly Gln Ile Thr Met Ile Asn Gly Ser Ile Arg Asp
        115                 120                 125

Ala Glu Thr Ala Val Ile Ser Glu Ser Thr Trp Thr Thr His Asn Pro
    130                 135                 140

Gln Ala Thr Asn Val Ile Leu Asn Asn Val Val Ile Glu Ala Lys Val
145                 150                 155                 160

Ala Leu Tyr Thr Lys Gly Pro Asn Ala Asn Ile Ile Met Thr Gly Gly
                165                 170                 175

Thr Ile Ala Ala Thr Gln Asn Ala Phe Thr Val Glu Asn Gly Gly Met
            180                 185                 190

Ile Arg Val Thr Asp Val Ser Ala Thr Ala Asp Glu Ile Gly Ile Ile
        195                 200                 205

Leu Ala Asn Thr Asn Asn Asp Lys Arg Pro Asn Arg Ile Asp Leu Thr
    210                 215                 220

Asn Thr Lys Leu Ile Val Glu Asn Gly Ile Gly Ile Ile Gly Ser Ala
225                 230                 235                 240

Glu Asn Ser Ile Ile Asn Leu Asn Asn Ser Glu Ile Arg Ala Asp Leu
                245                 250                 255

Leu Leu Asn Ser Ile Asn Val Asp Glu Asn Lys Asn Pro Ala Arg Leu
            260                 265                 270

Asn Leu Lys Ala Glu Asn Ser Ile Leu Glu Gly Asn Val Arg Ile Ala
        275                 280                 285

Ala Asn Asp Thr Val Ser Phe Asp Leu Thr Asn Thr Thr Trp Tyr Leu
    290                 295                 300

Lys Ile Ser Asn Glu Lys Asp Ala Asn Gly Asn Leu Leu Ser Ile Lys
305                 310                 315                 320

Asp Arg Ser His Ser Gln Leu Ser Thr Leu Lys Leu Lys Asp Ser Ser
                325                 330                 335

Ile Ile Phe Lys Glu Pro Thr Gly Asn Asn Tyr Gln Thr Leu Leu Ile
            340                 345                 350

Ser Ala Gln Arg Pro Ala Ser Phe Ile Glu Asp Gln Asp Leu Asn Gln
        355                 360                 365

Lys Asn Gln Thr Ala Ile Tyr Ser Ala Glu Gly Asn Thr Gln Ile His
    370                 375                 380

Phe Asn Thr Val Trp Ser Asp Gly Ala Lys Ile Thr Asp Gln Lys Thr
385                 390                 395                 400

Asp Arg Val Leu Ile His Gly Asp Val Ser Gly Thr Thr Met Ile His
                405                 410                 415

Val Asn Val Lys Glu Gly Asp Asn Val Gln Lys Asp Thr Ser Leu Pro
            420                 425                 430

Trp Asn Gln Arg Gly Val Ser Leu Ile Gln Val Ser Gly Ile Ala Asn
        435                 440                 445

Glu Asn Ser Phe Lys Leu Ala Asn Gly Tyr Thr Thr Leu Asn Gly Thr
    450                 455                 460

Pro Tyr Lys Tyr Ile Leu Tyr Ala Tyr Gly Lys Gly Ser Ser His Gly
465                 470                 475                 480

Gly Ala Ser Ala Gly Gln Asn Ile Val Ser Asp Gly Asp Asp Phe Trp
                485                 490                 495

Asp Phe Arg Leu Gln Gly Glu Tyr Leu Pro Pro Pro Pro Asp Pro Asn
            500                 505                 510

Pro Glu Gly Pro Asp Thr Gly Lys Pro Asp Thr Gly Gly Pro Asp Thr
        515                 520                 525

Gly Gly Pro Asp Thr Gly Glu Pro Asp Thr Gly Gly Pro Asp Thr Gly
    530                 535                 540

Glu Pro Asp Thr Gly Gly Pro Asp Thr Gly Glu Pro Asp Thr Gly Gly
545                 550                 555                 560

Pro Asp Thr Gly Gly Pro Asp Thr Gly Glu Pro Asp Thr Gly Gly Pro
                565                 570                 575

Asp Thr Gly Gly Ala Gly His Arg Trp Thr Cys Thr Ser Ser Cys Ser
            580                 585                 590

Thr Ser Ser Lys Leu Tyr Ser Ser Ala Gln Cys Ser Leu Leu His Trp
        595                 600                 605

Tyr His
    610

<210>  10
<211>  297
<212>  PRT
<213>  B. bacilliformis

<400>  10

Met Asn Tyr Gln Asn Ala Phe Leu Ala Asn Met Arg Ser Met Ser Leu
1               5                   10                  15

Phe Met Lys Gly Gln Arg Lys Asn Thr Phe Leu Ala Ser Thr Tyr Ser
            20                  25                  30

Asp Thr Thr Thr Phe Ser Ser Glu Arg Thr Ala Trp Glu Tyr Gly Tyr
        35                  40                  45

Gly Ala Asp Ile Gln Tyr Thr Ala Leu Gln Thr Gly Val Val Leu Glu
    50                  55                  60

Thr Leu Glu Ser Gln Asn Ile Thr Thr Asp Phe Gly Leu Leu Gly Thr
65                  70                  75                  80

Tyr Gly Gln Leu Phe Phe Thr Pro Lys Asn Met Ala Asp Ala Ser Lys
                85                  90                  95

Ser Thr Leu Asn Lys Leu Ser Leu Thr Ala Tyr Ser Ser Thr Gln His
            100                 105                 110

Asn Asn Gly Leu Tyr Ala Asp Ile Leu Leu Ser Tyr Gly Leu Ile Ser
        115                 120                 125

Gly Asn Ile Thr Asn Ala Leu Ile Gly Asn Thr Ala Gln Val Asp Gly
    130                 135                 140

Thr Gln Thr Leu Ser Leu Ser Ala Thr Ile Gly Lys Pro Leu Ala Ile
145                 150                 155                 160

Gly Ile Ala Asp Leu Thr Phe Glu Pro Gln Ala Gln Leu Ala Tyr Gln
                165                 170                 175

Arg Leu Met Phe Gly Thr Ile Ser Asp Ile Asp Gly Phe Lys Val Asp
            180                 185                 190

Ile Gly Ser Pro Glu Gln Trp Leu Met Arg Ile Gly Gly Arg Leu Thr
        195                 200                 205

Lys Thr Leu Thr Ser Leu Gln Gln Gly Lys Ala Val Ser Phe Tyr Ser
    210                 215                 220

Lys Leu Asp Leu Ile Lys Thr Phe Gly Asp Glu Gly Ser Ile Lys Ile
225                 230                 235                 240

Gly Asp Asp Phe Lys Arg Asp Pro Thr Gly Ser Phe Val Glu Gly Gly
                245                 250                 255

Leu Gly Val Asn Met Gln Leu Ser Ala Lys Phe Ser Leu Tyr Gly Asp
            260                 265                 270

Ile Ser His Arg Arg Lys Leu Gln Lys Ser Gly Ile Ser Gly Asn Ser
        275                 280                 285

Phe Ser Ala Gly Ile Arg Phe Arg Phe
    290                 295

<210> 11
<211> 1058
<212> PRT
<213> B. bacilliformis

<400> 11

Met Leu Lys Lys Asn Ile Leu Leu Cys Thr Val Ala Gly Thr Leu Leu
1               5                   10                  15

Cys Ser Asn Phe Asn Val Ala Phe Ala Ser Val Pro Val Asp Glu Ile
            20                  25                  30

Lys Pro Asn Ser Trp Glu Arg Ile Lys Thr Asp Val Arg Val Gln Gly
        35                  40                  45

Leu Tyr Gly Ala Asp Asp Pro Gly Arg Val Asp Phe Leu Asp Asp Gln
    50                  55                  60

Ala Asn Tyr Gly Trp Gly Ile Ala Lys Lys Ile Glu Lys Leu Gln Arg
65                  70                  75                  80

Asp Val Ser Ala Phe Ser Lys Gln Met Leu Asp Phe Glu His Gln Val
                85                  90                  95

Ser Asp Leu Ala Gln Gly Asn Glu Gly Lys Ala Leu Leu Arg Gln Glu
            100                 105                 110

Gln Asp Ser Met Lys Asn Thr Val Gln Ala Leu Gln Gly Arg Val Glu
        115                 120                 125

Tyr Leu Lys Thr Gln Leu Asp Ser Glu Leu Gln Lys Gln Asn Gly Gln
    130                 135                 140

Lys Asp Gln Leu Glu Gln Asn Lys Val Glu Val Asn Asn Phe Leu Ala
145                 150                 155                 160

Arg Val Lys Asp Phe Gln Asp Gln Ile Lys Trp Phe Gly Asp Val Leu
                165                 170                 175

Ala Ser Tyr Asp Ser Glu Ala Glu Leu Ala Gly Asn Pro Val Gly Asp
            180                 185                 190

Ser Ser Ile Val Val Ser Asn Val Glu Tyr Pro Gly Gln Ile Val Thr
        195                 200                 205

Ile Lys Asp Thr Lys Ile His Gly Glu Phe Pro Met Ile Val Ser Glu
    210                 215                 220

Gly Gly Thr Ile Tyr Gly Val Asn Val Asn Thr Ser Glu Glu Ala Met
225                 230                 235                 240

Val Ala Leu Ser Asn Leu Gly Gly Arg Phe Glu Leu Tyr Asn Ser Asp
                245                 250                 255

Ile Asn Val Ala Trp Arg Glu Ser Ala Gly Ile Val Phe Ala Ser Lys
            260                 265                 270

Asp Ala Asn Asp His Val Val Lys Leu Val Asn Thr Asn Ile His Val
        275                 280                 285

Glu Asp Gly Val Gly Ile Phe Gly Ser Thr Phe Gly Asp Val Thr Gly
    290                 295                 300

Gln Ile Ser Leu Glu Asn Ser Lys Ile Ser Ala Asp Ile Leu Val Lys
305                 310                 315                 320

Lys Glu Asp Ala Asn Gln Ser Pro Leu His Leu Thr Ile Thr Ala Asp

325                 330                 335

Asn Ser Val Leu Arg Gly Gly Ile Arg Thr Ala Leu Glu Glu Arg Ala
            340                 345                 350

Ser Val Arg Ala Val Leu Gly Glu Arg Glu Gly Val Arg Asp Pro Gln
        355                 360                 365

Asp Thr Gln Thr Asp Gln Gln Arg Gln Asp Val Leu Ser Leu Leu Leu
    370                 375                 380

Gln Arg Asn Arg Gly Gln Arg Val Gln Leu Glu Ala Gly Ala Leu Glu
385                 390                 395                 400

Val Gln Asn Asn Gln Gln Val Asn Gly Val Gln Gln Val Leu Gly Asn
                405                 410                 415

Pro Asp Ile Asn Val Gln Ala Gln Ala Asn Gln Asp Ser Thr Gln Val
            420                 425                 430

Ser Glu Ala Ser Thr Val Ala Leu Ala Val Ser Glu Ile Ser Gly Glu
        435                 440                 445

Pro Thr Ser Gln Pro Arg Leu Thr Val Trp Asp Ala Pro Gln Gly Val
    450                 455                 460

Thr Val Leu Gly Pro Arg Val Glu Leu Ala Pro Arg Gln Gln Gln Val
465                 470                 475                 480

Asp Pro Ser Thr Gln Thr Gly Ser Thr Ser Leu Ser Thr Val Asn Leu
                485                 490                 495

Asp Leu Arg Asn Asn Ser Lys Trp Tyr Leu Thr Phe Ser Lys His Glu
            500                 505                 510

Met Asp Asp Val Ser Leu Glu Ser Lys Asp Glu Thr Arg Ile Glu Asn
        515                 520                 525

Arg Lys Arg Arg Leu Ser Ser Asp Leu Ser Ser Leu Lys Val His Asp
    530                 535                 540

Ser Ser Val Ile Phe Val Glu Pro Lys Ser Ser Asn His Tyr Lys Thr
545                 550                 555                 560

Leu Tyr Val Gly Phe Asp Asn Pro Glu Arg Asn Val Tyr Val Ala Lys
                565                 570                 575

Gly Asp Ala Arg Leu Leu Ile Asn Ala Glu Leu Asp Asp Ser Lys Lys
        580                 585                 590

Ile Gly Asn Leu Lys Ser Asp Gln Val Ile Val Ser Gly Asn Val Ile
        595                 600                 605

Gly Asn Thr Thr Ile His Val Gln Asp Met Thr Asn Ser Glu Leu Val
    610                 615                 620

Lys Leu Pro Lys Ile Thr His Arg Pro Ser Ile Leu Ala Ala Ser Glu
625                 630                 635                 640

Gly Ile Ile Pro Leu Asn Glu Arg Gly Val Ser Val Val Gln Val Phe
                645                 650                 655

Gly Thr Ala Lys Glu Asp Ser Phe Lys Leu Ser Asp Pro Tyr Ile Thr
            660                 665                 670

Leu Gly Gly Ser Pro Tyr Lys Tyr Val Leu Lys Ala Tyr Gly Pro Gly
        675                 680                 685

Ser Gln His Gly Glu Ser Arg Glu Ala Asp Val Ser Ala Val Tyr Asp
    690                 695                 700

Leu Leu Thr Ala Asn Ser Ser Glu Ser Pro Ala Phe Trp Asp Tyr Arg
705                 710                 715                 720

Leu Gln Asn Glu Tyr Ile Asp Ser Asp Arg Tyr Val Ser Ala Ile Val
                725                 730                 735

Pro Gln Ala Ala Ser Tyr Leu Val Met Ser Asn Ala Val Phe Ser Ser
            740                 745                 750

Gly Leu Ala Asp Val Ile Asn His Asn Thr Leu Ile Thr Ser Met Gln
        755                 760                 765

Lys Asn Pro Val Glu Lys Lys Gly Glu Gln Phe Ala Ile Ser Leu Ser
    770                 775                 780

Ser Tyr Gly Asn Lys Leu Thr Leu Ser Ser Asp Arg Thr Pro Phe Asp
785                 790                 795                 800

Tyr Gly Tyr His Ala Asp Val Asn Tyr Gly Ala Leu Gln Ala Gly Leu
                805                 810                 815

Ser Leu Val Glu Trp Asp Asn Lys Asp Ser Ile Thr His Phe Gly Leu
            820                 825                 830

Leu Gly Asn Tyr Gly Arg Leu Phe Phe Thr Pro Lys Asp Val Pro Asp
        835                 840                 845

Ser Asp Glu Ser Asn Leu Asn Lys Trp Ser Val Ala Val Tyr Ser Ser
    850                 855                 860

Thr Gln His Tyr Asp Gly Phe Tyr Ala Asn Ala Leu Ile Ser Tyr Gly
865                 870                 875                 880

Met Met Lys Gly Thr Ile Thr Asn Ala Val Ile Gly Asp Thr Ala Thr
                885                 890                 895

Ile Asp Asp Ala Lys Ala Trp Asn Ile Ser Leu Ala Leu Gly Lys Lys
            900                 905                 910

Ile Asp Thr Glu Val Lys Gly Val Arg Phe Glu Pro Glu Ala Gln Leu
        915                 920                 925

Val Tyr Gln Gly Leu Asn Phe Glu Thr Ile Ser Asp Ile Asp Lys Phe
    930                 935                 940

Asp Val Asp Met Asp Asp Pro Arg Gln Trp Leu Val Arg Val Gly Gly
945                 950                 955                 960

Arg Val Ser Lys Ile Ile Pro Met Asn Asn Ile Ile Pro Met Gly Asn
                965                 970                 975

Lys Asn Asp Phe Val Ser Leu Tyr Thr Lys Leu His Leu Ile Lys Thr
            980                 985                 990

Phe Ser Asp Asn Ser Thr Ile Thr Met Gly Asp Gln Phe His Val Asp
        995                 1000                1005

Ser Val Gly Ser Ser Leu Glu Gly Gly Leu Gly Phe Val Ser Glu
    1010                1015                1020

Phe Ser Asn Ser Leu Ser Leu His Ser Glu Ile Ser Tyr Arg Cys
    1025                1030                1035

Ala Leu Gln Arg Ser Gly Thr Ser Gly Thr Ser Phe Ser Gly Gly
    1040                1045                1050

Ile Arg Tyr Arg Phe
    1055

<210> 12
<211> 240

<212>  PRT
<213>  B. bacilliformis

<400>  12

Met Lys Tyr Lys Asp Lys Gln Tyr Ser Arg Leu Tyr Leu Val Phe Gly
1               5                   10                  15

Ile Ile Phe Thr Ser Val Ile Val Thr Ser Cys Ser Tyr Asn Thr Lys
            20                  25                  30

Asp Phe Asn Ser Val Pro Asp Phe Ser Pro Val Arg Ala Asp Leu Gly
        35                  40                  45

Tyr Ala Pro Ser Arg Thr Ala Gln Thr Tyr Pro Leu Pro Pro Arg Glu
    50                  55                  60

Ser Asn Tyr Ser Leu Tyr Arg Ala Ser Gly Asn Ser Phe Phe Arg Asp
65                  70                  75                  80

Pro Arg Ala Met Gln Pro Gly Asp Val Leu Thr Val Gln Ile Ser Ile
                85                  90                  95

Asn Asp Arg Ala Ser Leu Asn Asn Lys Ser Asp Leu Lys Ser Asp Ser
            100                 105                 110

Ser Ser Lys Tyr Thr Ile Gly Thr Gly Tyr Ser Phe Met Asp Arg Ile
        115                 120                 125

Ala Gly Ser Leu Glu Gly Glu Ser Ser Asn Gln Ser Lys Gly Asp Gly
    130                 135                 140

Lys Ile Glu Arg Gln Glu Asn Ile Arg Leu Ser Val Ala Ala Ile Val
145                 150                 155                 160

Thr Asp Val Leu Pro Asn Gly Asn Leu Ile Ile Arg Gly Ser Gln Glu
                165                 170                 175

Val Arg Val Asn His Glu Leu Arg Ile Leu Asn Ile Ala Gly Val Val
            180                 185                 190

Arg Pro Arg Asp Ile Ser Gly Asn Asn Met Ile Glu Tyr Asp Lys Ile
        195                 200                 205

Ala Glu Ala Arg Ile Ser Tyr Gly Gly Arg Gly Arg Ile Ser Glu Ile
    210                 215                 220

Gln Gln Pro Pro Tyr Gly Gln Gln Leu Leu Asn Gln Ile Ser Pro Phe
225                 230                 235                 240

<210>  13
<211>  284
<212>  PRT
<213>  B. bacilliformis

<400>  13
   Met Lys Met Arg Tyr Leu Ser Val Leu Phe Gly Ile Thr Leu Gly Ser
   1               5                   10                  15
   Ser Ser Val Val Asn Ala Ala Asp Val Val Val Ile Asn Lys Thr Glu
               20                  25                  30
   Glu Glu Ala Pro Ala Ser Ser Ser Val Phe Thr Pro Ala Asp Leu Ser
           35                  40                  45
   Trp Thr Gly Val Tyr Leu Gly Gly Gln Ile Ser Gly Phe Ser Ser Lys
       50                  55                  60
   Val Asp Ile Asp Arg Tyr Ser Asp Asn Thr Trp Ser Ser Val Glu Lys
   65                  70                  75                  80
   Ser Leu Leu Pro Glu Ile Ser Gly Phe Ser Gly Gly Leu Tyr Val Gly
                   85                  90                  95
   Ser Asn Ile Asp Leu Gly Glu Gly Phe Ile Leu Gly Val Glu Thr Asp
               100                 105                 110
   Ala Leu Trp Ser Asn Thr Gly Asp Lys Lys Thr Leu Pro Thr Lys Lys
           115                 120                 125
   Val Gly Ala Ala His Arg Leu Glu Ala Ala Glu Arg Asn Arg Glu Asp
       130                 135                 140
   Glu Val Val Gln Glu Arg Arg Val Ala Ala Ala Asn Asn Ser Ala Lys
   145                 150                 155                 160
   Ala Pro Glu Ala Asn Val Arg Met Thr Gly Glu Tyr Ala Leu Arg Gln
                   165                 170                 175
   Gln Trp Ile Gly Ala Thr Arg Ala Arg Val Gly Phe Ala Val Glu Arg
               180                 185                 190
   Phe Met Pro Tyr Val Thr Gly Gly Leu Ala Tyr Gly Gln Leu Arg Gly
           195                 200                 205
   Val Asn Leu Leu Ala Leu Ala Thr Asn Asn Thr Glu Asn Ser Glu Lys
       210                 215                 220
   Glu Glu Lys Leu Met Val Gly Tyr Ala Val Gly Ala Gly Leu Asp Phe
   225                 230                 235                 240
   Ala Val Ser Ser Asn Ile Phe Leu Arg Thr Glu Tyr Arg Phe Ser Asp
                   245                 250                 255
   Leu Gly Lys Lys Lys Phe Ala Asn Glu Lys Ile Glu Val Asn Tyr Lys
               260                 265                 270
   Ile Asn Asp Phe Arg Val Gly Val Ala Tyr Lys Phe
           275                 280

<210>  14
<211>  300
<212>  PRT
<213>  B. bacilliformis

<400>  14

Met Asn Ile Lys Cys Leu Val Thr Ala Ser Val Cys Ala Leu Ile Ser
1               5                   10                  15

Ala Ser Ala Ala Gln Ala Ala Asp Val Met Ile Pro Gln Glu Ile Ser
            20                  25                  30

Pro Ile Ile Ser Ala Pro Thr Phe Ser Trp Thr Gly Leu Tyr Leu Gly
        35                  40                  45

Gly Gln Ile Gly Gly Leu Ser Gly Lys His Asp Phe Lys Ala Ile Gly
    50                  55                  60

Lys Asp Ser Glu Trp Pro Phe Ala Asn Lys Asp Leu Lys Val Ser Gly
65                  70                  75                  80

Phe Val Gly Gly Leu Tyr Ala Gly Ser Asn Ile Asp Leu Gly Ser Gly
                85                  90                  95

Leu Val Leu Gly Val Asp Thr Asp Ile Val Trp Val Asp Lys Glu Gly
            100                 105                 110

Lys Leu Ser Ser Asn His Lys Ala Glu Thr Gln Asp Asp Ala Asp Ala
        115                 120                 125

Phe Lys Gln Ile Phe Asp Glu Asn Lys Ile Glu Val Ala Lys Gly Gln
    130                 135                 140

Ile Lys Glu Leu Thr Gln Asn Phe Ser Leu Lys Glu Lys Trp Ala Gly
145                 150                 155                 160

Ala Thr Arg Val Arg Ile Gly Phe Gly Ala Asp Arg Ile Met Pro Tyr
                165                 170                 175

Val Ser Gly Gly Val Ala Tyr Thr Gln Val Gln Ala Ile Gly Ser Ala
            180                 185                 190

Ile Leu Lys Gly Thr Lys Asp Thr Gly Thr Glu Gly Gly Gly Gly Thr
        195                 200                 205

Glu Gly Gly Gly Gly Thr Glu Gly Gly Gly Gly Ser Ala Ser Lys Ala
    210                 215                 220

Val Arg Ser Glu Ala Leu Asp Val Leu Ala Ser Gly Thr Ile Thr Asp
225                 230                 235                 240

Glu Lys Lys Thr Leu Leu Gly Tyr Thr Leu Gly Ala Gly Val Asp Phe
                245                 250                 255

Ala Met Thr Asp Asn Val Ile Leu Arg Thr Glu Tyr Arg Tyr Ser Asp
            260                 265                 270

Phe Gly Lys Lys Lys Phe Val Lys Asp Ala Ile Glu Thr Asn Tyr Lys
        275                 280                 285

Thr Asn Asp Phe Arg Val Gly Val Ala Tyr Lys Phe
    290                 295                 300

## Description

### FIELD OF THE INVENTION

The invention is based on the identification of immunodominant proteins of *Bartonella bacilliformis.* The immunodominant proteins identified in context of the invention are used as protein and mRNA vaccines as well as in the development of immunological detection assays for the diagnosis of an infection with *Bartonella bacilliformis.*

### DESCRIPTION

Carrion's disease is a vector-borne biphasic illness restricted to the South American Andes with Peru as the most affected country (1). The causative agent of this neglected disease is *Bartonella bacilliformis,* which is transmitted to humans by *Lutzomyia* spp. sandflies (2). In the acute phase, *B. bacilliformis* infects human erythrocytes causing severe hemolytic anemia called "Oroya fever" with a case-fatality rate of up to 88% if untreated (3) often followed by a second and chronic phase known as "verruga peruana" resulting in endothelial proliferation (4).

Very little is known about the immune response upon *B. bacilliformis* infections with some evidence that humoral and cellular mechanisms are involved. It is known that an infection results in a lifelong humoral immunity which confers partial protection (5) and this is in line with earlier results showing that primates recovered from an infection showed complete immunity when repetitively infected (6).

The acute phase "Oroya fever" is mainly diagnosed based on clinical symptoms and by the detection of Giemsa-stained bacteria in peripheral blood smears (specificity: ∼96%, sensitivity ∼ 36%) (7). The use of microbiological cultures is limited due to the slow growth of *B. bacilliformis* (∼14 days) but several diagnostic PCR-based approaches have been established directly from peripheral blood (8). The number of asymptomatic *B. bacilliformis* human carriers was calculated with ∼37% in post-outbreak areas and ∼52% in endemic areas by qPCR. Asymptomatic individuals are considered to represent the main reservoir of the pathogen (9). However, PCR-based diagnostics require relatively high expertise and expensive equipment, are therefore not feasible in remote areas and do not allow to detect past infections.

The seroprevalence of *B. bacilliformis* antibodies in human sera is up to ∼65% in endemic areas of Peru (10). Anti-*B*. *bacilliformis* Pap31 IgM and IgG might represent suitable markers to detect recent infections and past exposure (9). Although efforts have been made to apply Pap31 to an ELISA-format (11), this test is not available yet. Furthermore, vaccines are not established until today although urgently needed (12).

Thus, it is an objection of the invention to provide novel sources for the development of vaccines against *B. bacilliformis* as well as immunological means for the detection of infections.

### BRIEF DESCRIPTION OF THE INVENTION

In this invention, the systematic identification of immunodominant proteins of *B. bacilliformis* for establishing a reliable serodiagnostic tool is provided. In total, 14 immunoreactive proteins were detected by a hybrid approach using reverse vaccinology and genomic *B. bacilliformis* expression libraries. Seroreactivity of the identified proteins was evaluated by using line blots and subsequent ELISA based on promising antigen candidates (porin B, autotransporter E and hypothetical protein B). Generally, and by way of brief description, the main aspects of the present invention can be described as follows:

In **a first aspect,** the invention pertains to a method for inducing an immune response against *Bartonella bacilliformis* in a subject, the method comprising the steps of
- Providing an immunological composition comprising at least one isolated immunodominant protein selected from a protein shown in any one of SEQ ID NO: 1-14, or comprising an isolated protein fragment having at least 8 consecutive amino acids of an amino acid sequence shown in any of SEQ ID NO: 1-14, or providing a nucleic acid sequence encoding the immunodominant protein or protein fragment thereof;
- Administering the immunological composition to the subject in an amount sufficient to induce an immune response.

In **a second aspect,** the invention pertains to a method for producing a vaccine composition for vaccinating a subject against Bartonella bacilliformis, the method comprising the steps of
- Providing at least one isolated immunodominant protein selected from a protein shown in any one of SEQ ID NO: 1-14, or comprising an isolated protein fragment having at least 8 consecutive amino acids of an amino acid sequence shown in any of SEQ ID NO: 1-14; or providing a nucleic acid sequence encoding the immunodominant protein or protein fragment thereof,
- Formulating the immunodominant protein or fragment thereof of (i) with at least one carrier and/or excipient suitable for use in a vaccine composition, such as a pharmaceutically acceptable carrier and/or excipient.

In **a third aspect,** the invention pertains to a method of detecting a *Bartonella* bacilliformis-infected subject, said method comprising detecting in a biological sample from the subject at least one protein selected from a group comprising SEQ ID NO: 1-14 and conservative variants thereof, or antibodies against at least one protein selected from a group comprising SEQ ID NO: 1-14 and conservative variants thereof in an immunoassay, wherein detection of said protein or said antibodies indicates that the subject is infected and not vaccinated.

In **a fourth aspect,** the invention pertains an antibody or antibody-serum produced with a method of the herein disclosed invention.

In **a fifth aspect,** the invention pertains to a lateral flow immunoassay device for detecting *Bartonella bacilliformis* -infected subjects, comprising:
- a sample pad configured for receiving a biological sample;
- a conjugate pad comprising conjugated antibodies raised against a protein selected from a group comprising SEQ ID NO: 1-14 and conservative variants thereof;
- a lateral flow membrane comprising a test line and a control line, wherein the test line comprises antibodies raised against a protein selected from a group comprising SEQ ID NO: 1-14 and conservative variants thereof, and the control line; and
- an absorbent pad.

In **a sixth aspect,** the invention pertains to a lateral flow immunoassay device for detecting *Bartonella bacilliformis* -infected subjects, comprising:
- a sample pad configured for receiving a biological sample;
- a conjugate pad comprising conjugated antibody;
- a lateral flow membrane comprising a test line and a control line, wherein the test line comprises antigens comprising the amino acid sequence selected from a group comprising SEQ ID NO: 1-14 and conservative variants thereof, and the control line comprises unrelated proteins and lipo-poly saccharides; and
- an absorbent pad.

In **a seventh aspect,** the invention pertains to a use of a protein selected from a group comprising SEQ ID NO: 1-14, or conservative variants and combinations thereof for detecting *Bartonella bacilliformis* in a subject.

In **an eighth aspect,** the invention pertains to an immunoassay kit for detecting *Bartonella bacilliformis* in a subject, comprising a solid support immobilized with primary antibodies raised against a protein selected from a group comprising SEQ ID NO: 1-14 and conservative variants thereof.

In **a ninth aspect,** the invention pertains to an immunoassay kit for detecting *Bartonella bacilliformis* in a subject, comprising a solid support immobilized with a protein selected from a group comprising SEQ ID NO: 1-14 and conservative variants thereof.

In **a tenth aspect,** the invention pertains to a vaccine composition, comprising
- at least one isolated immunodominant protein selected from a protein shown in any one of SEQ ID NO: 1-14, or comprising an isolated protein fragment having at least 8 consecutive amino acids of an amino acid sequence shown in any of SEQ ID NO: 1-14; and/or
- an expressible nucleic acid comprising a nucleic acid sequence coding for at least one isolated immunodominant protein selected from a protein shown in any one of SEQ ID NO: 1-14, or coding for an isolated protein fragment having at least 8 consecutive amino acids of an amino acid sequence shown in any of SEQ ID NO: 1-14;
together with a pharmaceutically acceptable carrier and/or excipient.

### DETAILED DESCRIPTION OF THE INVENTION

In the following, the elements of the invention will be described. These elements are listed with specific embodiments, however, it should be understood that they may be combined in any manner and in any number to create additional embodiments. The variously described examples and preferred embodiments should not be construed to limit the present invention to only the explicitly described embodiments. This description should be understood to support and encompass embodiments which combine two or more of the explicitly described embodiments or which combine the one or more of the explicitly described embodiments with any number of the disclosed and/or preferred elements. Furthermore, any permutations and combinations of all described elements in this application should be considered disclosed by the description of the present application unless the context indicates otherwise.

The term "immunodominant protein" or "immunogenic peptide" as used herein also includes peptides and polypeptides that are immunologically active in the sense that once administered to a host, it is able to evoke an immune response of the humoral and/or cellular type directed against the protein. Preferably the protein fragment is such that it has substantially the same immunological activity as the total protein. Thus, a protein or polypeptide fragment according to the invention comprises or consists essentially of or consists of at least one epitope or antigenic determinant. The term epitope, also known as antigenic determinant, is the part of a macromolecule recognized by the immune system and able to induce an immune reaction of the humoral type (B cells) and/or cellular type (T cells).

The term "immunodominant protein" or "immunogenic peptide" further contemplates deletions, additions and substitutions to the sequence, so long as the polypeptide functions to produce an immunological response as defined herein. In this regard, particularly preferred substitutions will generally be conservative in nature, i.e., those substitutions that take place within a family of amino acids. For example, amino acids are generally divided into four families: (1) acidic-aspartate and glutamate; (2) basic-lysine, arginine, histidine; (3) non-polar-alanine, valine, leucine, isoleucine, proline, phenylalanine, methionine, tryptophan; and (4) uncharged polar-glycine, asparagine, glutamine, cysteine, serine threonine, tyrosine. Phenylalanine, tryptophan, and tyrosine are sometimes classified as aromatic amino acids. It is reasonably predictable that an isolated replacement of leucine with isoleucine or valine, or vice versa; an aspartate with a glutamate or vice versa; a threonine with a serine or vice versa; or a similar conservative replacement of an amino acid with a structurally related amino acid, will not have a major effect on the biological activity. Proteins having substantially the same amino acid sequence as the reference molecule but possessing minor amino acid substitutions that do not substantially affect the immunogenicity of the protein are, therefore, within the definition of the reference polypeptide. In preferred embodiments an immunodominant protein of the invention is a protein as shown in any one of SEQ ID NO: 1 to 14.

The term "isolated" immunodominant protein, "isolated" polypeptide, or "isolated peptide" is a protein, polypeptide or peptide that by virtue of its origin or source of derivation (1) is not associated with naturally associated components that accompany it in its native state, (2) is free of other proteins from the same species, (3) is expressed by a cell from a different species, or (4) does not occur in nature. Thus, a peptide that is chemically synthesized or synthesized in a cellular system different from the cell from which it naturally originates will be "isolated" from its naturally associated components. A protein may also be rendered substantially free of naturally associated components by isolation, using protein purification techniques well known in the art.

In one embodiment, the peptide consists of, consists essentially of, or comprises an amino acid sequence selected from the group consisting of SEQ ID Nos: 1 to 14 and functionally active variants thereof. In another aspect, said peptide consists of, consists essentially of, or comprises an amino acid sequence selected from the group consisting of SEQ ID Nos: 1 to 14 and functionally active variants thereof.

In another embodiment, the peptide consists of, or consists essentially of, an amino acid sequence selected from the group consisting of SEQ ID Nos: 1, 2, 3, 4, 5, 6, 7, 8, 9, 10, 11, 12, 13, and/or 14. In an aspect, the polypeptide includes an amino acid sequence at least 80%, 81%, 82%, 83%, 84%, 85%, 86%, 87%, 88%, 89%, 90%, 91%, 92%, 93%, 94%, 95%, 96%, 97%, 98%, or 99% identical to the amino acid sequence set forth in SEQ ID NO: 1, SEQ ID NO:2, SEQ ID NO:3, SEQ ID NO:4, SEQ ID NO:5, SEQ ID NO:6, SEQ ID NO:7, SEQ ID NO:8, SEQ ID NO:9, SEQ ID NO:10, SEQ ID NO:11, SEQ ID NO:12, SEQ ID NO:13, and/or SEQ ID NO:14. In an aspect, the polypeptide includes a contiguous amino acid sequence at least 80%, 81%, 82%, 83%, 84%, 85%, 86%, 87%, 88%, 89%, 90%, 91%, 92%, 93%, 94%, 95%, 96%, 97%, 98%, or 99% identical to the contiguous amino acid sequence set forth in EQ ID NO: 1, SEQ ID NO:2, SEQ ID NO:3, SEQ ID NO:4, SEQ ID NO:5, SEQ ID NO:6, SEQ ID NO:7, SEQ ID NO: 8, SEQ ID NO:9, SEQ ID NO:10, SEQ ID NO:11, SEQ ID NO:12, SEQ ID NO:13, and/or SEQ ID NO:14.. In an aspect, the polypeptide includes an amino acid sequence having at least 10, 15, 20, 25, 30, 35, 40, 45, 50, 55, 60, 65, 70, 75, 80, 85, 90, 95, 100, 200, 300, 400, or 500 (or any integer within these numbers) contiguous amino acids of the amino acid sequence set forth in SEQ ID NO: 1, SEQ ID NO:2, SEQ ID NO:3, SEQ ID NO:4, SEQ ID NO:5, SEQ ID NO:6, SEQ ID NO:7, SEQ ID NO:8, SEQ ID NO:9, SEQ ID NO:10, SEQ ID NO:11, SEQ ID NO:12, SEQ ID NO:13, and/or SEQ ID NO:14. Examples of proteins with 90-95% identity to one or more of SEQ ID NOs 1-14 are shown below in **Table 1.**

The term "epitope" is the part of a macromolecule recognized by the immune system and able to induce an immune reaction of the humoral type (B cells) and/or cellular type (T cells). The term is also used interchangeably with "antigenic determinant" or "antigenic determinant site". Antibodies that recognize the same epitope can be identified in a simple immunoassay showing the ability of one antibody to block the binding of another antibody to a target antigen.

An "immunological response" to a composition or vaccine is the development in the host of a cellular and/or antibody-mediated immune response to a composition or vaccine of interest. Usually, an "immunological response" includes but is not limited to one or more of the following effects: the production of antibodies, B cells, helper T cells, and/or cytotoxic T cells, directed specifically to an antigen or antigens included in the composition or vaccine of interest. Preferably, the host will display either a therapeutic or protective immunological response such that resistance to new infection will be enhanced and/or the clinical severity of the disease reduced. Such protection will be demonstrated by either a reduction or lack of symptoms normally displayed by an infected host, a quicker recovery time and/or a lowered viral titer in the infected host.

The term "immunogenic" or "immunodominant" protein or polypeptide as used herein also refers to an amino acid sequence which elicits an immunological response as described above. An "immunogenic" protein or polypeptide, as used herein, includes the full-length sequence of the protein, analogs thereof, or immunogenic fragments thereof. By "immunogenic fragment" is meant a fragment of a protein which includes one or more epitopes and thus elicits the immunological response described above. Such fragments can be identified using any number of epitope mapping techniques, well known in the art. See, e.g., Epitope Mapping Protocols in Methods in Molecular Biology, Vol. 66 (Glenn E. Morris, Ed., 1996). For example, linear epitopes may be determined by e.g., concurrently synthesizing large numbers of peptides on solid supports, the peptides corresponding to portions of the protein molecule, and reacting the peptides with antibodies while the peptides are still attached to the supports. Such techniques are known in the art and described in, e.g., U.S. Pat. No. 4,708,871; Geysen et al., 1984; Geysen et al., 1986. Similarly, conformational epitopes are readily identified by determining spatial conformation of amino acids such as by, e.g., x-ray crystallography and 2-dimensional nuclear magnetic resonance. See, e.g., Epitope Mapping Protocols, supra.

Synthetic antigens are also included within the definition, for example, polyepitopes, flanking epitopes, and other recombinant or synthetically derived antigens. Immunogenic fragments, for purposes of the present invention, will usually include at least about 3 amino acids, about 5 amino acids, about 10-15 amino acids, about 15-25 amino acids or more amino acids, of the molecule. There is no critical upper limit to the length of the fragment, which could comprise nearly the full-length of the protein sequence, or even a fusion protein comprising at least one epitope of the protein.

Accordingly, a minimum structure of a polynucleotide expressing an epitope is that it comprises or consists essentially of or consists of nucleotides to encode an epitope or antigenic determinant of an immunodominant protein or polypeptide in accordance with the herein described invention, preferably a protein of any one of SEQ ID NO:1 to 14. A polynucleotide encoding a fragment of the total protein or polypeptide comprises or consists essentially of or consists of a minimum of 15 nucleotides, advantageously about 30-45 nucleotides, and preferably about 45-75, at least 57, 87 or 150 consecutive or contiguous nucleotides of the sequence encoding the total protein or polypeptide. Epitope determination procedures, such as, generating overlapping peptide libraries (Hemmer et al., 1998), Pepscan (Geysen et al., 1984; Geysen et al., 1985; Van der Zee R. et al., 1989; Geysen, 1990; Multipin^{©} Peptide Synthesis Kits de Chiron) and algorithms (De Groot et al., 1999), can be used in the practice of the invention, without undue experimentation.

A "polynucleotide" is a polymeric form of nucleotides of any length that contains deoxyribonucleotides, ribonucleotides, and analogs in any combination. Polynucleotides may have three-dimensional structure, and may perform any function, known or unknown. The term "polynucleotide" includes double-, single-, and triple-stranded helical molecules. Unless otherwise specified or required, any embodiment of the invention described herein that is a polynucleotide encompasses both the double stranded form and each of two complementary forms known or predicted to make up the double stranded form of either the DNA, RNA or hybrid molecule.

The following are non-limiting examples of polynucleotides: a gene or gene fragment, exons, introns, mRNA, tRNA, rRNA, siRNA, ribozymes, cDNA, recombinant polynucleotides, branched polynucleotides, plasmids, vectors, isolated DNA of any sequence, isolated RNA of any sequence, nucleic acid probes and primers. A polynucleotide may comprise modified nucleotides, such as methylated nucleotides and nucleotide analogs, uracil, other sugars and linking groups such as fluororibose and thiolate, and nucleotide branches. The sequence of nucleotides may be further modified after polymerization, such as by conjugation, with a labeling component. Other types of modifications included in this definition are caps, substitution of one or more of the naturally occurring nucleotides with an analog, and introduction of means for attaching the polynucleotide to proteins, metal ions, labeling components, other polynucleotides or solid support. The polynucleotides can be obtained by chemical synthesis or derived from a microorganism.

The term "gene" is used broadly to refer to any segment of polynucleotide associated with a biological function. Thus, genes include introns and exons as in genomic sequence, or just the coding sequences as in cDNAs and/or the regulatory sequences required for their expression. For example, gene also refers to a nucleic acid fragment that expresses mRNA or functional RNA, or encodes a specific protein, and which includes regulatory sequences.

The invention further comprises a complementary strand to a polynucleotide encoding a *Bartonella bacilliformis* protein, antigen, epitope or immunogen. The complementary strand can be polymeric and of any length, and can contain deoxyribonucleotides, ribonucleotides, and analogs in any combination thereof.

The terms "protein", "peptide", "polypeptide" and "polypeptide fragment" are used interchangeably herein to refer to polymers of amino acid residues of any length. The polymer can be linear or branched, it may comprise modified amino acids or amino acid analogs, and it may be interrupted by chemical moieties other than amino acids. The terms also encompass an amino acid polymer that has been modified naturally or by intervention; for example disulfide bond formation, glycosylation, lipidation, acetylation, phosphorylation, or any other manipulation or modification, such as conjugation with a labeling or bioactive component.

An "isolated" polynucleotide or polypeptide is one that is substantially free of the materials with which it is associated in its native environment. By substantially free, is meant at least 50%, at least 70%, at least 80%, at least 90%, or at least 95% free of these materials.

Hybridization reactions can be performed under conditions of different stringency. Conditions that increase stringency of a hybridization reaction are well known. See for example, "Molecular Cloning: A Laboratory Manual", second edition (Sambrook et al., 1989). Examples of relevant conditions include (in order of increasing stringency): incubation temperatures of 25° C., 37° C., 50° C., and 68° C.; buffer concentrations of 10×SSC, 6×SSC, 1×SSC, 0.1×SSC (where SSC is 0.15 M NaCl and 15 mM citrate buffer) and their equivalent using other buffer systems; formamide concentrations of 0%, 25%, 50%, and 75%; incubation times from 5 minutes to 24 hours; 1,2 or more washing steps; wash incubation times of 1,2, or 15 minutes; and wash solutions of 6×SSC, 1×SSC, 0.1×SSC, or deionized water.

The invention further encompasses polynucleotides encoding functionally equivalent variants and derivatives of the *Bartonella bacilliformis* polypeptides and functionally equivalent fragments thereof that may enhance, decrease or not significantly affect inherent properties of the polypeptides encoded thereby. These functionally equivalent variants, derivatives, and fragments display the ability to retain the activity. For instance, changes in a DNA sequence that do not change the encoded amino acid sequence, as well as those that result in conservative substitutions of amino acid residues, one or a few amino acid deletions or additions, and substitution of amino acid residues by amino acid analogs are those which will not significantly affect properties of the encoded polypeptide. In one embodiment, the variants have at least 50%, at least 55%, at least 60%, at least 65%, at least 70%, at least 75%, at least 80%, at least 85%, at least 86%, at least 87%, at least 88%, at least 89%, at least 90%, at least 91%, at least 92%, at least 93%, at least 94%, at least 95%, at least 96%, at least 97%, at least 98% or at least 99% homology or identity to the *Bartonella bacilliformis* polynucleotide or polypeptide of interest.

In the **first aspect,** the invention pertains to a method for inducing an immune response against *Bartonella bacilliformis* in a subject, the method comprising the steps of
- Providing an immunological composition comprising at least one isolated immunodominant protein selected from a protein shown in any one of SEQ ID NO:1-14, or comprising an isolated protein fragment having at least 8 consecutive amino acids of an amino acid sequence shown in any of SEQ ID NO: 1-14, or providing a nucleic acid sequence encoding the immunodominant protein or protein fragment thereof;
- Administering the immunological composition to the subject in an amount sufficient to induce an immune response.

Preferred is that the immunological composition does not comprise *Bartonella bacilliformis* complete cells (organisms) or any fragments thereof that are not an immunodominant protein or fragment thereof as mentioned above.

The invention may provide either therapeutic methods or non-therapeutic methods. In the former case, the invention pertains to medical applications such as vaccinations or treatments of diseases associated with the infection of a subject with *Bartonella bacilliformis.* The latter case may refer to methods for the generation of or the testing of an immune response, for example in context of antibody generation. In this case preferably non-human animals (vertebrates) a subject of a non-therapeutic method for the generation of antibodies specific for the immunodominant protein or fragment thereof. In this context, the method of the first aspect may include a further step of isolating antibody producing cells from the subject, optionally of immortalizing the cells and obtaining antibodies therefrom. Such methods are well known to the person of skill in the relevant art.

In **a second aspect,** the invention pertains to a method for producing a vaccine composition for vaccinating a subject against *Bartonella bacilliformis,* the method comprising the steps of
- Providing at least one isolated immunodominant protein selected from a protein shown in any one of SEQ ID NO: 1-14, or comprising an isolated protein fragment having at least 8 consecutive amino acids of an amino acid sequence shown in any of SEQ ID NO: 1-14; or providing a nucleic acid sequence encoding the immunodominant protein or protein fragment thereof,
- Formulating the immunodominant protein or fragment thereof of (i) with at least one carrier and/or excipient suitable for use in a vaccine composition, such as a pharmaceutically acceptable carrier and/or excipient.

With regard to the second aspect, the invention in some embodiments provides the method as an in vitro and/or non-therapeutic method.

In the **third aspect,** the invention pertains to a method of detecting a *Bartonella* bacilliformis-infected subject, said method comprising detecting in a biological sample from the subject at least one protein selected from a group comprising SEQ ID NO: 1-14 and conservative variants thereof, or antibodies against at least one protein selected from a group comprising SEQ ID NO: 1-14 and conservative variants thereof in an immunoassay, wherein detection of said protein or said antibodies indicates that the subject is infected and not vaccinated.

Where said immunoassay is an immune affinity procedure, said procedure is any one of enzyme-linked immunosorbent assay (ELISA), Western Blot, immuno-precipitation, FACS, Lateral Flow, Biochip arrays (Lim et al., 2005; Ewalt et al., 2001). An immunoassay may involve the detection of primary antibodies or secondary (labelled) antibodies conjugated to any signalling label such as a fluorescence substance such as (but not limited to) those incorporating lanthanides, compatible with detection by Time-Resolved Fluorometry (TRF, which is based on the use of antibodies conjugated to a fluorescence substance exhibiting a large Stoke shift and a slow decay of the emitted fluorescence which may be consequently measured at high resolution) using any suitable fluorescence detection device (Peruski et al., 2002). Immunoassay may be any assay which is based on ECL (Electrochemical luminescence) involving the use of antibodies or antibody fragments conjugated to substances such as (but not limited to) ruthenium, where detection is mediated by any suitable device (such as the BioVeris platform, Rivera et al., 2006). Such detection modalities were described before and are known to persons skilled in the field of immunodetection (for example, Lim et al., 2005). Preferred secondary antibodies are conjugated with a colorimetric, chemiluinescent, fluorescent, enzyme or gold- nanoparticle label.

In particular embodiments of the invention the method comprises
- contacting a biological sample with immobilized primary antibodies on the solid support, wherein the primary antibodies are specific to SEQ ID NO: 1-14 and conservative variants thereof and the primary antibodies form an antibody-antigen complex with SEQ ID NO: 1-14 and conservative variants thereof; and
- detecting the presence of antigens bound to the primary antibodies using conjugated secondary antibodies raised against a protein selected from a group comprising SEQ ID NO: 1-14 and conservative variants thereof, wherein the detection of said antigens indicates that the subject is infected and not vaccinated with Bartonella bacilliformis.

In a particular alternative embodiment, the method comprises
- contacting a biological sample with the immobilized antigenic immunodominant protein selected from a group comprising SEQ ID NO: 1-14 and conservative variants thereof on the solid support, wherein the immobilized antigenic immunodominant protein form an antigen- antibody complex with antibodies specific to said antigenic proteins; and
- detecting the presence of antibodies bound to the antigenic immunodominant protein using conjugated secondary antibodies, wherein the detection of said antibodies indicates that the animal is infected and not vaccinated with Bartonella bacilliformis.

The term "sample" in the present specification and claims is used herein in its broadest sense. It is meant to include both biological and environmental samples. Harvesting of these samples may be for (without being restricted to) diagnostic, forensic or epidemiologic purposes. A sample may include a specimen of natural or synthetic origin. Biological samples may be obtained from: an animal, particularly mammals including humans, a fluid, a solid (e.g., stool) or a tissue, as well as liquid and solid food and feed products and ingredients such as dairy items, vegetables, meat and meat by-products, and waste. The term "sample" may also include body fluids (urine, blood, etc.), swabs taken from suspected body regions (throat, vagina, ear, eye, skin, sores), food products (both solids and fluids), swabs (taken from medicinal instruments, apparatus, materials), samples taken from air (for airborne bacteria especially from ventilation systems), samples taken from the environment (soil, water, plant parts). Typically swabs and samples that are a priori not liquid are contacted with a liquid medium which is then contacted with the detecting agent. Any said environment sample may serve as an inoculum for a bacterial culture which will represent the examined sample. Preferred are biological samples that are a liquid sample, such as a blood sample, saliva sample, urine sample, such as a blood derived sample selected from a serum or plasma sample.

In **the fourth aspect,** the invention pertains an antibody or antibody-serum produced with a method of the herein disclosed invention.

The term "antibody" as used herein in all aspects and embodiments refers to an immunoglobulin molecule or immunologically active portion thereof, i.e., an antigen-binding portion. Examples of immunologically active portions of immunoglobulin molecules include F(ab) and F(ab')2 fragments, which retain the ability to bind their specific epitope. Such fragments can be obtained commercially or using methods known in the art. For example F(ab)2 fragments can be generated by treating the antibody with an enzyme such as pepsin, a nonspecific endopeptidase that normally produces one F(ab)2 fragment and numerous small peptides of the Fc portion.

As used herein, the term antibody is intended to include intact molecules, such as a naturally occurring, synthetic or recombinant antibody, a chimeric antibody, as well as fragments thereof, such as for example, scFv, Fv, Fab', Fab, diabody, linear antibody or F(ab')2 antigen binding fragment of an antibody which are capable of binding the antigen. The generation of polyclonal or monoclonal antibodies is well known to the skilled artisan.

Monoclonal antibodies may be prepared from B cells taken from the spleen or lymph nodes of immunized animals, in particular rats or mice, by fusion with immortalized B cells under conditions which favour the growth of hybrid cells. The technique of generating monoclonal antibodies is described in many articles and textbooks, such as the above-noted Chapter 2 of Current Protocols in Immunology. Spleen or lymph node cells of these animals may be used in the same way as spleen or lymph node cells of protein-immunized animals, for the generation of monoclonal antibodies as described in Chapter 2 therein. The techniques used in generating monoclonal antibodies are further described in by Kohler and Milstein [Kohler and Milstein (1975) Nature 256; 495-497] and in U.S. Pat. No. 4,376,110.

Fab and F(ab')2 and other fragments of antibodies are typically produced by proteolytic cleavage, using enzymes such as papain (to produce Fab fragments) or pepsin (to produce F(ab')2 fragments).

Further to the herein described *Bartonella bacilliformis* diagnostic, the biomarkers described herein, in particular the proteins of SEQ ID NO: 1 to 14, may serve for further monitoring of subjects that may or may not be previously identified as infected with *Bartonella bacilliformis,* in order to confirm previous exposure. The identification or diagnosis of previous *Bartonella bacilliformis* infection may be through detection of said biomarkers in samples obtained from said subjects or, alternatively, through the detection of circulating antibodies against said biomarkers (clearly at a stage further to seroconversion) in such infected subjects. Another alternative scenario is that of subjects who may have been treated for bacterial infection, even without specific diagnosis of *Bartonella bacilliformis,* and thus a specific diagnostic of *Bartonella bacilliformis* may be desirable, for example for epidemiological purposes.

In **the fifth aspect,** the invention pertains to a lateral flow immunoassay device for detecting *Bartonella bacilliformis* -infected subjects, comprising:
- a sample pad configured for receiving a biological sample;
- a conjugate pad comprising conjugated antibodies raised against a protein selected from a group comprising SEQ ID NO: 1-14 and conservative variants thereof;
- a lateral flow membrane comprising a test line and a control line, wherein the test line comprises antibodies raised against a protein selected from a group comprising SEQ ID NO: 1-14 and conservative variants thereof, and the control line; and
- an absorbent pad.

In **the sixth aspect,** the invention pertains to a lateral flow immunoassay device for detecting *Bartonella bacilliformis* -infected subjects, comprising:
- a sample pad configured for receiving a biological sample;
- a conjugate pad comprising conjugated antibody;
- a lateral flow membrane comprising a test line and a control line, wherein the test line comprises antigens comprising the amino acid sequence selected from a group comprising SEQ ID NO: 1-14 and conservative variants thereof, and the control line comprises unrelated proteins and lipo-poly saccharides; and
- an absorbent pad.

Lateral flow immunoassays (LFAs) can be used for diagnosing diseases at point-of-care. LFAs are intended to detect the presence (or absence) of a target analyte in liquid sample (matrix) without the need for specialized and costly equipment, though many lab-based applications exist that are supported by reading equipment. Typically, LFAs are used for medical diagnostics either for home testing, point of care testing, or laboratory use. A widely spread and well known application is the home pregnancy test. A typical LFA comprise a series of capillary beds having the capacity to transport fluid (e.g. a biomarker such as blood, saliva or urine) spontaneously. A first capillary bed comprises a sample pad acting as a sponge to hold an excess of sample fluid. Once the first capillary bed is soaked, the sample fluid migrates to a second capillary bed in the form of a conjugate pad in which the manufacturer has stored the so-called conjugate, a dried format of bio-active particles in a salt-sugar matrix, that is selected to provide a chemical reaction between the target molecule (e.g. an antigen) and its chemical partner (e.g. an antibody) that has been immobilized on the particle's surface. The analyte binds to the particles while migrating further through a third capillary bed having one or more areas (often called"stripes" or a "test line") where a third molecule has been immobilized by the manufacturer. By the time the sample-conjugate mix reaches these stripes, analyte has been bound on the particle and the third "capture" molecule binds the complex. After a while, when more and more fluid has passed the stripes, particles accumulate and the stripes change colour to provide a visual indicator which can be used to deduce the identity and/or concentration of the analyte. After passing these reaction zones, the fluid enters the final porous material, comprising a wick, that simply acts as a waste container. The test strip itself is typically contained within a casing/holder.

In particular embodiments, the antibody in the conjugate pad is conjugated with a colorimetric, chemiluminescent, fluorescent, enzyme or gold nanoparticle label.

In some embodiments the lateral flow membrane is a nitrocellulose membrane.

In **a seventh aspect,** the invention pertains to a use of a protein selected from a group comprising SEQ ID NO: 1-14, or conservative variants and combinations thereof for detecting *Bartonella bacilliformis* in a subject.

In **an eighth aspect,** the invention pertains to an immunoassay kit for detecting *Bartonella bacilliformis* in a subject, comprising a solid support immobilized with primary antibodies raised against a protein selected from a group comprising SEQ ID NO: 1-14 and conservative variants thereof.

In **a ninth aspect,** the invention pertains to an immunoassay kit for detecting *Bartonella bacilliformis,* preferably antibodies against *Bartonella bacilliformis*,in a subject, comprising a solid support immobilized with a protein selected from a group comprising SEQ ID NO: 1-14 and conservative variants thereof.

In **a tenth aspect,** the invention pertains to a vaccine composition, comprising
- at least one isolated immunodominant protein selected from a protein shown in any one of SEQ ID NO: 1-14, or comprising an isolated protein fragment having at least 8 consecutive amino acids of an amino acid sequence shown in any of SEQ ID NO: 1-14; and/or
- an expressible nucleic acid comprising a nucleic acid sequence coding for at least one isolated immunodominant protein selected from a protein shown in any one of SEQ ID NO: 1-14, or coding for an isolated protein fragment having at least 8 consecutive amino acids of an amino acid sequence shown in any of SEQ ID NO: 1-14;
together with a pharmaceutically acceptable carrier and/or excipient.

In certain specific embodiments and aspects, the induction of immune response for the purpose of vaccination, treatment or antibody generation, may be affected by the use of an RNA vaccine composition. The RNA (e.g., mRNA) vaccines may be utilized in various settings depending on the prevalence of the infection or the degree or level of unmet medical need. In some instances, the RNA (e.g., mRNA) vaccines have superior properties in that they produce much larger antibody titers and produce responses earlier than commercially available anti-viral therapeutic treatments. While not wishing to be bound by theory, it is believed that the RNA (e.g., mRNA) vaccines, as mRNA polynucleotides, are better designed to produce the appropriate protein conformation upon translation as the RNA (e.g., mRNA) vaccines co-opt natural cellular machinery. Unlike traditional vaccines, which are manufactured ex vivo and may trigger unwanted cellular responses, RNA (e.g., mRNA) vaccines are presented to the cellular system in a more native fashion. Hence, in some aspects the invention is a vaccine, comprising at least one RNA polynucleotide having an open reading frame encoding at least one *Bartonella bacilliformis* antigenic polypeptide, formulated in a cationic lipid nanoparticle, preferably wherein the antigenic polypeptide is selected from an immunodominant protein of any one of SEQ ID NO:1 to 14, or an immunogenic fragment thereof.

Some embodiments of the present disclosure provide RNA (e.g., mRNA) vaccines that include at least one RNA (e.g., mRNA) polynucleotide having an open reading frame encoding at least one antigenic polypeptide or an immunogenic fragment thereof (e.g., an immunodominant protein) and at least one RNA (e.g., mRNA polynucleotide) having an open reading frame encoding an adjuvant, such as a flagellin adjuvant.

Provided herein, in some embodiments, is a ribonucleic acid (RNA) (e.g., mRNA) vaccine, comprising at least one (e.g., at least 2, 3, 4 or 5) RNA (e.g., mRNA) polynucleotide having an open reading frame encoding at least one (e.g., at least 2, 3, 4 or 5) antigenic polypeptide selected from SEQ ID NO: 1 to 14, or any combination of two or more of the foregoing antigenic polypeptides. Herein, use of the term "antigenic polypeptide" encompasses immunogenic fragments of the antigenic polypeptide (an immunogenic fragment that is induces (or is capable of inducing) an immune response to *Bartonella bacilliformis),* unless otherwise stated.

The terms "of the [present] invention", "in accordance with the invention", "according to the invention" and the like, as used herein are intended to refer to all aspects and embodiments of the invention described and/or claimed herein.

As used herein, the term "comprising" is to be construed as encompassing both "including" and "consisting of', both meanings being specifically intended, and hence individually disclosed embodiments in accordance with the present invention. Where used herein, "and/or" is to be taken as specific disclosure of each of the two specified features or components with or without the other. For example, "A and/or B" is to be taken as specific disclosure of each of (i) A, (ii) B and (iii) A and B, just as if each is set out individually herein. In the context of the present invention, the terms "about" and "approximately" denote an interval of accuracy that the person skilled in the art will understand to still ensure the technical effect of the feature in question. The term typically indicates deviation from the indicated numerical value by ±20%, ±15%, ±10%, and for example ±5%. As will be appreciated by the person of ordinary skill, the specific such deviation for a numerical value for a given technical effect will depend on the nature of the technical effect. For example, a natural or biological technical effect may generally have a larger such deviation than one for a man-made or engineering technical effect. As will be appreciated by the person of ordinary skill, the specific such deviation for a numerical value for a given technical effect will depend on the nature of the technical effect. For example, a natural or biological technical effect may generally have a larger such deviation than one for a man-made or engineering technical effect. Where an indefinite or definite article is used when referring to a singular noun, e.g. "a", "an" or "the", this includes a plural of that noun unless something else is specifically stated.

It is to be understood that application of the teachings of the present invention to a specific problem or environment, and the inclusion of variations of the present invention or additional features thereto (such as further aspects and embodiments), will be within the capabilities of one having ordinary skill in the art in light of the teachings contained herein.

Unless context dictates otherwise, the descriptions and definitions of the features set out above are not limited to any particular aspect or embodiment of the invention and apply equally to all aspects and embodiments which are described.

All references, patents, and publications cited herein are hereby incorporated by reference in their entirety.

### BRIEF DESCRIPTION OF THE FIGURES AND SEQUENCES

The figures show:

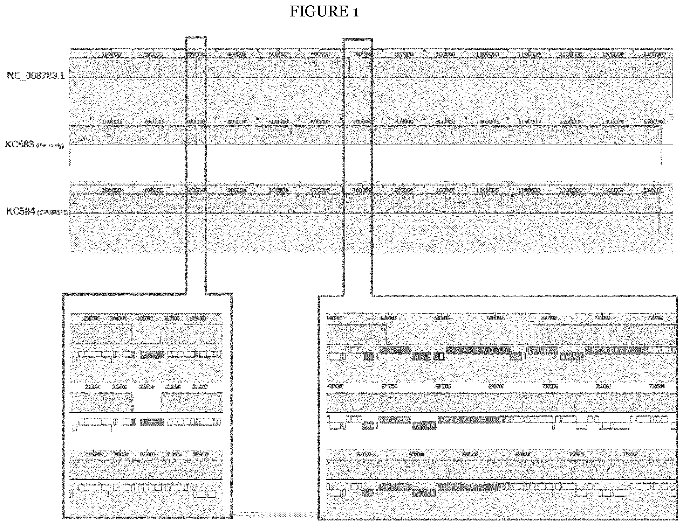

**Figure 1****:** shows a genomic alignment of three *B. bacilliformis* genomes using MAUVE. Shown are the genome of the isolate used in this study (termed 'KC583'), the genome of strain KC584 (CP045671.1) and the earlier KC583 sequence (NC_008783.1). Boxes provide a close-up view on differing regions affecting more than on gene. In the left box, the genes BARBAKC583_RS01505, BARBAKC583_RS01510 and BARBAKC583_RS01515 all absent for the strain KC584 are colored in pink. In the right box, the genes of a repetitive region are colored in orange and blue for NC_008783.1 while for KC583 and KC584 only one set of these genes is present.

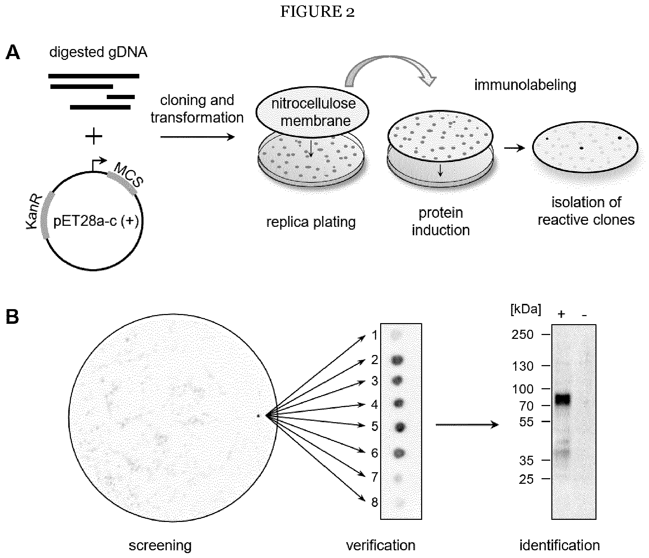

**Figure 2****:** shows the generation of heterologous *B. bacilliformis* expression libraries. (A) Schematic work flow. Fragments of partially digested genomic *B. bacilliformis* KC583 DNA were cloned into pET28a/b/c protein expression vectors. E. coli BL21 was transformed with the resulting plasmid libraries, cultured on LB agar (incl. kanamycin) and transferred to nitrocellulose membranes. Protein production and cell lysis were performed directly on the membranes. Immunoreactive proteins were detected using sera of an immunized rabbit or of infected Peruvian patients. (B) Example of a nitrocellulose membrane screening disc. One reactive colony (black colored spot) was detected and eight subclones were re-tested with patient sera to verify reactivity and purity. The reactive subclone (here: #5) was further analyzed by conventional western blotting and plasmid sequencing (data not shown). Here, a 75kDa fragment of autotransporter E was detected from the ITPG-induced culture whereas no signal was detected from the non-induced culture (-).

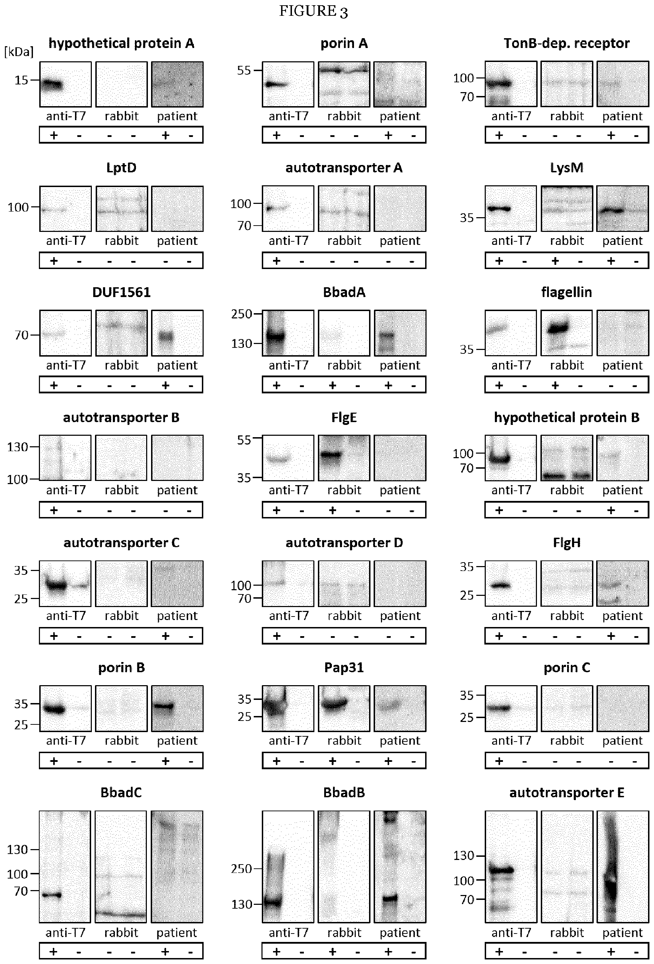

**Figure 3****:** shows a Western blot analysis of target proteins. All target proteins (see figure subheadings) were produced in E. coli BL21 (+) and subjected to immunoblotting. For this purpose, heterologous protein production was confirmed by T7 tag western blotting (anti-T7; technical control). For analysis of immunoreactivity, serum of a B. bacilliformis-immunized rabbit (technical control) and a pool of five IFA and western blot positive Peruvian patient sera were used. Reactivity is given individually per target protein [bottom rows, indicated as "+" (reactive) and "-" (non-reactive)]. Negative control: non induced protein expression lysates.

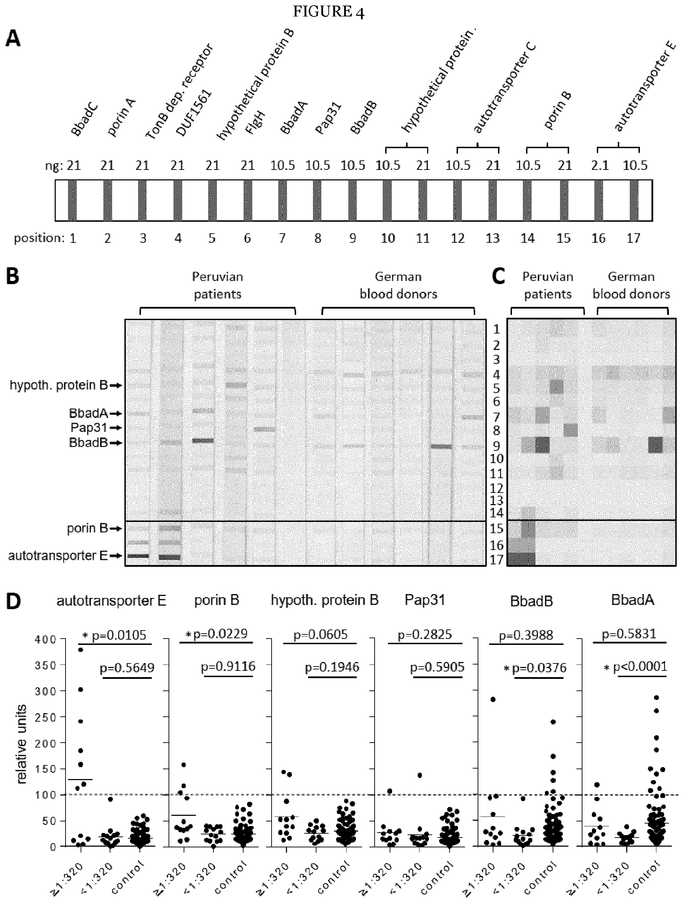

**Figure 4****:** shows a line blot analysis of Peruvian patient and control sera using *B. bacilliformis* seroreactive proteins. (A) Design of the line blot. His-tag purified antigens were printed on nitrocellulose membranes (2.1 ng, 10.5 ng, 21 ng per antigen band). (B) Line blot analysis. Line blots were incubated either with human (IFA-titer ≥1:320, n=6, left) or control sera of German healthy blood donors (n=6, right). The most reactive proteins are marked with arrows. (C) Densitometric analysis of reactivity. Visualization of protein reactivity by heat maps. Values are shown ranging from no binding (light blue) to maximum binding (dark blue). Numbers refer to the position of each protein on the line blot. (D) Analysis of antigen reactivity using line blots. Results for the most reactive antigens (BbadA, BbadB, Pap31, hypothetical protein B, porin B, autotransporter E) are presented for three groups of sera: (i) sera with a high anti-*B*. *bacilliformis* IFA-titer from patients suffering from a *B. bacilliformis* infection (n=i2; ≥1:320), (ii) sera with an IFA titer <1:320 (n=14) and control sera (n=96). Values higher than a band intensity of 100 units are considered positive. Respective P values between sample categories are given (* p <0.05).

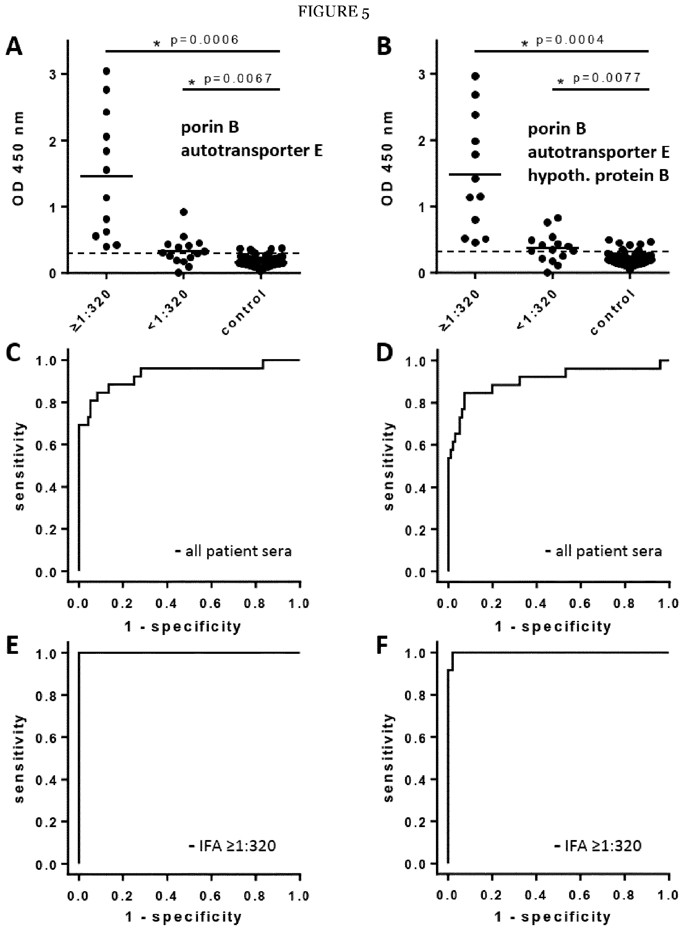

**Figure 5****:** shows an analysis of Peruvian patient sera and control sera using *B. bacilliformis* ELISAs. (A,B) ELISA analysis. Antigen composition: porin B, autotransporter E (left), porin B, autotransporter E, hypothetical protein B (right). Sera with high anti-*B*. *bacilliformis* IFA-titers from patients suffering from a *B. bacilliformis* infection (n=12; ≥1:320), (ii) sera with an IFA titer <1:320 (n=14) and control sera (n=96) were used. Values higher than OD450 0,29 (A) and 0,34 (B) were considered positive. Respective P-values between sample categories are given (* p <0.05). (C,D) ROC curve using all Peruvian patient sera (n=26) and (E,F) ROC curve using Peruvian patient sera with an IFA-titer ≥1:320 (n=12). Values are depicted in a ROC diagram according to sensitivity (true-positive rate) and 1 - specificity (false-positive rate).

The sequences show:

SEQ ID NO: 1 - BARBAKC583_00274 (BbadC)

MKEIQPQRGAVGCVLVSYFWLKRFLGIAIAALLSTILPAEGQPIEHVTLSEHNGVPIVDG
AFELQQSSNSGDRDCRNMNEVINRLSREVGDGRVLPLELEEKYCETIVERLHNLGDSNGISLQA
LNMIEGTGVHKGNSGKFKVYSVGLGHYSGVLGVGAATAVNAFGAQAYGFGARAMAPATIAVGI
GADAQNMGAVAVGGIASAKGKNSLALGPQTLAYSDGSVAIGGDPDSEFGARALATNSLSMGAK
SYTGENSEAAIAVGLGAQVRDGSPGGVALGWGAFVAEGAEGGLALGRNSHVDVNQGVALGVG
SRVNTPAGVAGYDPRSNDETKNTNSAWKSRYGAVSVGDAERDITRQITGVAAGTQNTDAVNVA
QLQALRDATVLYNRDEDGNKTNKIALQGGDPSQPVALDNLADGNVTEGSKQAVNGGQLYDFM
QVQTDYTKEQTRRVLKDAKGYTDKKFNALSYEIDGARKEARQGAAIGLAVSNLRYDDTPGSLSL
AFGTGMWRHQYGFAVGAGFMSVDGKVSANVSAATAGGHWGVGAGVSVTLN

SEQ ID NO: 2 - BARBAKC583_00276 (BbadB)

MKKNSTTKGNNLDAKGNDLDDCHFPYRSFLFKAVILGTAMTALLSSVALVFPVHSASIE
ETAQNAKKSESLKDYRSQNKKGADTSHLWGLNKNIALMDGVAQAKHKTLNVAAPTRNLLLKE
NITIGSLMNAADRMATDSISIMHSNYEGRDAPINCAISFDTPTAMCFGFRERMDSQDLPSISLA
HYAHSYDNRVSDNYGSQIDKASKEGDVQSDSSRSLSKEAEGEEKTYLEEGAKLADILNDTPIVT
TFMTQDKPNLGGGDVFISANNSSGDKIYSRFGIAETRSLDDLLCNTASKDILCIGQLTAAKGFAS
VALGYGSQAIRGPNVAGYDPITDGISTSSDYLWKSTHGELGIGNMDIKRSRQITGVAAGREDNE
AVNVAQLKALRKWVQTRDTDISDIIQFRLLNVYGSGQISIGWDKASVQSISVNHKNGNKRYIEG
IRDGVIASNSSMAVTGGQLYSAYKTVEKFLGGGASFGVGGFGDKQPIFNIQGNRYRDVGAAFDG
VDNSFSKFDKSIEEIKNDISSAMGDSLVKQDVKTGIITIGSEKEGTVIDIANSKQETRQLTGLKAG
IISQNSTDAVTGGQLWTTREKVNALDTNIKVAILDIAIVKNDVSILETNVNNAAANISQYLGSYA
DVLKGVAPIYEIQGQTYRDVGAAFAGVDDSFGKFDKSIEEIKNDISSAMGDSLVKQDVKTGIITI
GSEKEGTVIDIANSKQETRQLTGLKAGIISQNSTDAVTGGQLWTTREKVNALDTNIKAAVLDIAI
LEGDVSILETNVNNAAANISQYLGGYADVLKGVAPIYEIQGQTYRDISSAFDGVDKFLTKINSGN
LEQDALLWSDEENAFVALHAAEKGKKSQNSKLKFLLDGDVSEGSTEAITGNQLYVVSNQLALY
LGGGAGYNNGQWIAPTFKVAQFSNNGSYSAEKTYNNVADAFDGVNKSMADINNRVNEATQN
GGKDSLNWNENIGTFDASHNGKPGAVTNVADGKVEQGSKDAINGGQLWDVKEDVKGVQNQV
NDLKDKVNGFDDKLNGIENAVDDMKDKIKDATDGVVSYDKDKNGKKTNKITLQGGDESKPVT
LDNVADGKVEKGSQEAVNGGQLHDYTKEKMQLVLDDAKKYTDEKIKNIVVDFVDEAVAKAKD
YTDQKFNVLSYEIDGARKEARQGAAIGLAVSNLRYDDTPGKFSLAFGAGMWRGESAVALGSAY
TSENGRVRSNLSATSGGGHWGVGAGLSLTLN

SEQ ID NO: 3 - BARBAKC583_00298 (hypothetical protein A)

MKHIYSISCLTITALLLAACSFSQYYGQYPFVRNIPNGIDGEWIDTNGIVSSFHNGIFETR

AADTEEKLSEGIYNYVNTQHVEIEIHSILRGTISRADCTISNNAMQLFCTSSNGLQFSLNRKI

SEQ ID NO: 4 - BARBAKC583_00404 (porin A)

MNIKTLLLCSTVAFIGISGAHAAPDIVEKPKSIKYLRACDAYGAGYFYIPGTETCMKLSGY

VRASFKGGHDVDAKTNADLDQHKKTYDVSSRLALIFQAASETELGTLRSYARLTSDWVNGKSG

AGGRLNAAYIRLGGFSVGLDDTIFSTWTSYGEVINDDLIAPAGNVRTNFVSYTFASNTGFAAIIG
AELGNASDPIGPDKKYYYIDKSDQVSIVADNDLLSKQTKDYIPHILLGAQFVQGWGGVSTITVYD
SHYKKWANKTRLDFKVNDDINVWVTGGYKTNVDYYTADEQNVLSRENTTIYAGWGGKWAAW
TGVTYQITPEAKLNTQISYSAVKTFSASANIVYTLVPGFTITPEVSYIAWKDDRTFKGQQDSAQRN
STPNYTHALNGKNALQTMIRLQRSF

SEQ ID NO: 5 - BARBAKC583_00413 (TonB-dependent
hemoglobin/transferrin/lactoferrin family receptor)
MQIKWKNIYKSCVALSALSIWAPSFVFAQNNDEGSVTDLKSIVIEGKQDLDPSSTVTILT
DRQTAQNIEQKQVSDVHDVNRLNSSISYNSSNDSFIVRGLDANRVLTTIDGIPLPWLNDEARSV
KGGNSTFDFNTLSTFDLIQGSDSSIYGSGALGGIIALRTLNPEDLLTEEKNWGSLTKGSYNSVDN
SWQVDQAYAVRANQTFVLFQGSLVGGHERKNMGSIGDYGTKRTLANPADFDQNNLLFKVHQ
YFDHNHRLSFTAERFHYNKDTHVMNAPAIYFPESVHEENKKSRERFSLSYDYEGSQDTLLDAF
HGQIYWQNQLNNHILSGNRVVSPKGNYLRDNQIRNTTYGLNTNSIKKVDIGTTTNTLQLAINIF
ESKFHQYASGKDNCHLPENARGCAFLHTNQSDAPDTNSRGFGLAFENEIDFYRYHVRITPGIRY
DWYTHNPQETASYGKVPISQKLPSKSSSHFSPKLRLEWDIHNQVALYAQWAQAFRAPSVSELY
LNYINPSFYYRAGNPDLKPETSNGYDLGVKYGNMNFGGSFGAFINDYKNFIDLVDKGPSREFRL
SREHYVNRAHVRIFGVETKAHWNLDNGLHSDFSLSYAHGKDLDKKEYLSSIPALKTIFGLGYAK
EFWGLDFVLTLAAKNDKVAKKSSYREVPGYSLVDVLSWWKPFGKDGLTIRAGVYNIFNTKYWN
ASDLPSVSPRGESPLPKDYFSQPGRSFKVSFVQKF

SEQ ID NO: 6 - BARBAKC583_00473 (LysM peptidoglycan-binding domain-containing
protein)

MYLKTLNDIFRYNFQKIALLAIATIITGCSSGTQRFFDGFYEGEASTQLTENTAMSADRRI
ISQSSVIQSDELPPVEPLHSSGDHNHASYNFSRQEAGSSFRENRVSPDGRIMGSPPQNLGTLSRS
NVNNPSIFQKGSYIVQSGDTLFSIAQQKGISVESLKVANGMGDNAIYIGQKLVIPDKQTVVTSNA
QNNAVGSSTTTSSTQSKGMSFVKKEEISAASRSVNTAPAAIINTPDVKKNVSKQMTTSKSASNSS
KTVKDSDNITVPQATGVSKMRWPVRGRLLSHFGQKKGTITNRGIDINVPEGSSVKAAENGVVIY
ASDGLKELGNVVMIRHEDNIITIYGHNSKLTVSRGQRVKRGDEIAKSGVSGDATTPRVYFEIRQ
NSVPVNPAQYLEN

SEQ ID NO: 7 - BARBAKC583_00801 (DUF1561 family protein)

MKLKLFFFFLSLLLTIQTSSAAPVPGVIQKPPDRPHDKAIKIRVHTGGEYCYTPTFTEGM
GYIYINDCSSSTVKARYDLFQRVAWNINDTWLCMTAPESITGSDSNSSANWDYLTLKPCVINDQ
NQRWIIKDGGFYTADERFRVKDTHWYAYISKDSKASYDHQLTFEMKDWSSTVSPPGNISFKTY
VAWLYVSVYPPLFALYYLQNDSSSISTTPLPLYYNPENGHIAQYYDSYGSFYCMASKQTTSQDWN
WVQWTECTDKIPSKKDSTFWDVSQLNGNEGFILDYQGNPLRVTQYGTRWGVPYTAKTSYIPKD
TTNSPTSKFILSTDIEQWNRYTNGNLGETLPFCPAPGKKGSILQTSQARKGLFLPPWFTLSEAWI
QRLWQIARSTVDGTERIGFCGICMLQTSQMLAELQENYQTPTYQDGAGYFFNTAFGRDPFVSL

RSRFPHIAERLQMTEVYSETPFTPTEDRHERANRACNVMISTFLPDVMWRSSAVGRASAEIART
LIGLFRIPPGTIGFVEVVRENTERTGYVAHAQPFIRTQEGIILIPTNAPQLSLDEFRQSLTPIPTTQ
QGLERLVLYLSVRGSQPIRSVSTFEMVQLTLPSLNVYMSQRDCTGEGDHRRGSRQLPSTSSINE
CASGGGRCSPR

SEQ ID NO: 8 - BARBAKC583_00820 (BbadA)

MKKLYLIPEENDLNCHHSLRCSPLVKAVSLGTVMAALLSSVSPVFAANVAILGSNVQSS
NDGSIPYSNGSHGSIVLAGDNNWCGVDNVTDRGKHLPQSSRAVKEISAEEQYERFVNNGEFFN
RHPYGVTTEQVKWEADGVTSSNGGYMGSSTTGGHSNVMPTAFGVYSFATGCGSYASGNYSTAF
GAGATTNGGGAQAYGVSALAKGQATIAMGVGAEAWDTSAVAVGGRAVALGKNSVALGTKAKA
SGDYGVALGTEALAKQRGSIAIGGDIPQEENDQGKRFGAMVEAENAIAIGVRSYASEDSKEAVAI
GLSAQVRDEAGIALGRGAVVRQGGSAGVAIGASAHVIADTGVALGTASIASTEHNVSGYNPATG
ATTKSSDHVWVSTRGAVSIGDVSQKVTRQITGVAAGSEMTDAVNVAQLKALRETIDKGWMLTV
NGADATPVGADGLDFSVENPNLDTPNLSIVRSGNKLTFDLAEHIQVGRITTGLSSLSNAGLVIKG
CNDGTACKNGPSVTAGGINAGDKTLTGVADAVLDNDAVNFSQLKALKETVDKGWMLTVNGSD
ATPVGADGADFSVGSSNLVITKDNKNKLTFDLAKDINVNSIKVGKISLDNNSVMIEGGPQISAD
GADFASKNIRGVADGVLDGDAVNFSQLKALKETVDKGWMLTVNGADATPVGADGADFSVGSS
NLVITKDNKNKLTFDLAEHIEVGRITTGLSSLSNAGLVIKGCNDGTACKNGPSVTAGGINAGDK
TLTGLADGVLDGDAVNVAQLNKVKEAIKGSGLIQQGENEDENGRITIGAGNSGTEINILNKDGV
RRTISGISGGKVTEESTEAINGAQLWETNEEVHNLNHRVDETHTNVSQYFGGGADISSGKAPTY
TIEGKQYNNVGSAFSAVNDSLDNIQNEISRHFGQIYTISDIFSHYFGGGAGYNNGKWTAPTYEVS
HFGTNGTSSEKAYNNVADAFGGVNESMSGMNDRLQEVENKVDQNKAESDSLGWSKDKGAYD
ASHDGKPSKIVNVADGQVAQGSEDAVNGGQLWETNERVTNVENKVNDVSKQVDSIENTVNHF
ADGAVSYQRDESGKKTNKVVLQGGDSGKPVTIDNVADGKIEKGSQEAVNGGQLHDYTQEQMK
LVLNDAKKYTDGKIKNIVVNAIDDAVSQAKGYTDMKFEALSYGIEGARKEARQAAAIGLAVSNL
RYNDTPGRLSFAFGSGMWRSQSAFALGTGYTSEDGKIQSNFSATTAGGHWGIGAGVSITLN

SEQ ID NO: 9 - BARBAKC583_01021 (hypothetical protein B)

MYKKYLLSCTTAVSFLLLGFHLDANAQRIVIDDGRTENISDLTLSAVSPRPEHGGVYVTD
GTLNGSDLIIFGTAGNGYGATADGNNAITHNGGKSIIQFQDTLIRYVTVGLFAKDRGQITMINGS
IRDAETAVISESTWTTHNPQATNVILNNVVIEAKVALYTKGPNANIIMTGGTIAATQNAFTVENG
GMIRVTDVSATADEIGIILANTNNDKRPNRIDLTNTKLIVENGIGIIGSAENSIINLNNSEIRADLL
LNSINVDENKNPARLNLKAENSILEGNVRIAANDTVSFDLTNTTWYLKISNEKDANGNLLSIKD
RSHSQLSTLKLKDSSIIFKEPTGNNYQTLLISAQRPASFIEDQDLNQKNQTAIYSAEGNTQIHFNT
VWSDGAKITDQKTDRVLIHGDVSGTTMIHVNVKEGDNVQKDTSLPWNQRGVSLIQVSGIANE
NSFKLANGYTTLNGTPYKYILYAYGKGSSHGGASAGQNIVSDGDDFWDFRLQGEYLPPPPDPNP
EGPDTGKPDTGGPDTGGPDTGEPDTGGPDTGEPDTGGPDTGEPDTGGPDTGGPDTGEPDTGG
PDTGGAGHRWTCTSSCSTSSKLYSSAQCSLLHWYH

SEQ ID NO: 10 - BARBAKC583_01022 (autotransporter C)

MNYQNAFLANMRSMSLFMKGQRKNTFLASTYSDTTTFSSERTAWEYGYGADIQYTALQ
TGVVLETLESQNITTDFGLLGTYGQLFFTPKNMADASKSTLNKLSLTAYSSTQHNNGLYADILLS
YGLISGNITNALIGNTAQVDGTQTLSLSATIGKPLAIGIADLTFEPQAQLAYQRLMFGTISDIDGF
KVDIGSPEQWLMRIGGRLTKTLTSLQQGKAVSFYSKLDLIKTFGDEGSIKIGDDFKRDPTGSFVE
GGLGVNMQLSAKFSLYGDISHRRKLQKSGISGNSFSAGIRFRF

SEQ ID NO: 11 - BARBAKC583_01024 (autotransporter E)

MLKKNILLCTVAGTLLCSNFNVAFASVPVDEIKPNSWERIKTDVRVQGLYGADDPGRVD
FLDDQANYGWGIAKKIEKLQRDVSAFSKQMLDFEHQVSDLAQGNEGKALLRQEQDSMKNTV
QALQGRVEYLKTQLDSELQKQNGQKDQLEQNKVEVNNFLARVKDFQDQIKWFGDVLASYDSE
AELAGNPVGDSSIVVSNVEYPGQIVTIKDTKIHGEFPMIVSEGGTIYGVNVNTSEEAMVALSNLG
GRFELYNSDINVAWRESAGIVFASKDANDHVVKLVNTNIHVEDGVGIFGSTFGDVTGQISLENS
KISADILVKKEDANQSPLHLTITADNSVLRGGIRTALEERASVRAVLGEREGVRDPQDTQTDQQ
RQDVLSLLLQRNRGQRVQLEAGALEVQNNQQVNGVQQVLGNPDINVQAQANQDSTQVSEAST
VALAVSEISGEPTSQPRLTVWDAPQGVTVLGPRVELAPRQQQVDPSTQTGSTSLSTVNLDLRNN
SKWYLTFSKHEMDDVSLESKDETRIENRKRRLSSDLSSLKVHDSSVIFVEPKSSNHYKTLYVGF
DNPERNVYVAKGDARLLINAELDDSKKIGNLKSDQVIVSGNVIGNTTIHVQDMTNSELVKLPKI
THRPSILAASEGIIPLNERGVSVVQVFGTAKEDSFKLSDPYITLGGSPYKYVLKAYGPGSQHGESR
EADVSAVYDLLTANSSESPAFWDYRLQNEYIDSDRYVSAIVPQAASYLVMSNAVFSSGLADVINH
NTLITSMQKNPVEKKGEQFAISLSSYGNKLTLSSDRTPFDYGYHADVNYGALQAGLSLVEWDN
KDSITHFGLLGNYGRLFFTPKDVPDSDESNLNKWSVAVYSSTQHYDGFYANALISYGMMKGTIT
NAVIGDTATIDDAKAWNISLALGKKIDTEVKGVRFEPEAQLVYQGLNFETISDIDKFDVDMDDP
RQWLVRVGGRVSKIIPMNNIIPMGNKNDFVSLYTKLHLIKTFSDNSTITMGDQFHVDSVGSSLE
GGLGFVSEFSNSLSLHSEISYRCALQRSGTSGTSFSGGIRYRF

SEQ ID NO: 12 - BARBAKC583_01028 (FlgH)

MKYKDKQYSRLYLVFGIIFTSVIVTSCSYNTKDFNSVPDFSPVRADLGYAPSRTAQTYPLP
PRESNYSLYRASGNSFFRDPRAMQPGDVLTVQISINDRASLNNKSDLKSDSSSKYTIGTGYSFMD
RIAGSLEGESSNQSKGDGKIERQENIRLSVAAIVTDVLPNGNLIIRGSQEVRVNHELRILNIAGVV
RPRDISGNNMIEYDKIAEARISYGGRGRISEIQQPPYGQQLLNQISPF

SEQ ID NO: 13 - BARBAKC583_01101 (porin B)

MKMRYLSVLFGITLGSSSVVNAADVVVINKTEEEAPASSSVFTPADLSWTGVYLGGQISG
FSSKVDIDRYSDNTWSSVEKSLLPEISGFSGGLYVGSNIDLGEGFILGVETDALWSNTGDKKTLP
TKKVGAAHRLEAAERNREDEVVQERRVAAANNSAKAPEANVRMTGEYALRQQWIGATRARV
GFAVERFMPYVTGGLAYGQLRGVNLLALATNNTENSEKEEKLMVGYAVGAGLDFAVSSNIFLR
TEYRFSDLGKKKFANEKIEVNYKINDFRVGVAYKF

SEQ ID NO: 14 - BARBAKC583_01102 (porin family protein Pap31)

MNIKCLVTASVCALISASAAQAADVMIPQEISPIISAPTFSWTGLYLGGQIGGLSGKHDFK
AIGKDSEWPFANKDLKVSGFVGGLYAGSNIDLGSGLVLGVDTDIVWVDKEGKLSSNHKAETQD
DADAFKQIFDENKIEVAKGQIKELTQNFSLKEKWAGATRVRIGFGADRIMPYVSGGVAYTQVQA
IGSAILKGTKDTGTEGGGGTEGGGGTEGGGGSASKAVRSEALDVLASGTITDEKKTLLGYTLGA
GVDFAMTDNVILRTEYRYSDFGKKKFVKDAIETNYKTNDFRVGVAYKF

### EXAMPLES

Certain aspects and embodiments of the invention will now be illustrated by way of example and with reference to the description, figures and tables set out herein. Such examples of the methods, uses and other aspects of the present invention are representative only, and should not be taken to limit the scope of the present invention to only such representative examples.

### The examples show:

### Example 1: Genomic comparison of B. bacilliformis strains KC583 and KC584

A genome sequence (obtained by Illumina sequencing) of *B. bacilliformis* KC583 was earlier uploaded in GenBank (NC_008783.1). The inventors aimed to use comparable KC583 and KC584 sequences to predict immunoreactive proteins and to exclude strain specific results. Therefore, an in-depth genomic characterization of the *B. bacilliformis* isolate KC583 via de novo assembly was performed. Results prompted that the assembly was considerably shorter (1.417.364 bp) than the earlier NC_008783.1 sequence (1.445.021 bp). To identify what accounts for that difference, a genomic alignment using MAUVE was utilized. To this end, the NC_008783.1 sequence, the herein performed de novo long read assembly and the previously published genome of KC584 were compared. Results demonstrated that a major repetitive sequence of about 30 kb was absent for both the current KC 583 assemblies and from the KC584 genome (Figure 1). Compared to KC583, strain KC584 experienced a deletion in a second site resulting in a loss of BARBAKC583_RS1510 (bbadB) and a fusion of the adjacent genes BARBAKC583_RS1505 and BARBAKC583_RS1515. A disruptive in frame insertion of 30 bp was found for comEC (BARBAKC583_RS02895) and single-point mutation in lrp/asnC family transcriptional regulator (BARBAKC583_RS04165) was identified. Additional 21 proteins including TonB-dependent receptor were affected ranging from single point mutations to frameshift mutations.

### Example 2: Identification of potential immunodominant B. bacilliformis proteins via reverse vaccinology

Reverse vaccinology has been proven extremely useful for the identification of immunodominant vaccine candidates (24, 25). As only limited knowledge for *B. bacilliformis* has been experimentally gained (9), the inventors applied for a broader method for prediction of potential immunodominant target proteins by using a dynamic Vaxign target analysis for *B. bacilliformis* KC583 and KC584 including 1,209 and 1,200 protein sequences, respectively. As a result, 20 identical proteins were found (Table 1) with 15 presumptive outer membrane proteins and five extracellular proteins. Protein length ranged from 124 aa (hypothetical protein A) to 1.259 aa (BbadA) and adhesion probability from 0.401 (flagellin) to 0.872 (hypothetical protein B). Interestingly, BbadB was not identified by this approach because the software failed to predict the subcellular protein location.

**Table 3: Immunodominant protein candidates of B. bacilliformis. The 22 listed proteins were identified in silico by reverse vaccinology ^{(a)} or experimentally by immunoscreening of genomic B. bacilliformis expression libraries ^{(b)}. Additionally, BbadB ^{(c)} was selected due to its high homology to B. henselae BadA. Accession number, cellular localization, adhesion probability, number of transmembrane helices and protein length are given for each protein.**

| **#** | **accession numbers** | **note** | **OM: outer membrane EC: extracellular probability** | **adhesin probability** | **trans-membr ane helices** | **length** |
|---|---|---|---|---|---|---|
| **1^{a}** | WP_00576 5706.1 | AprI/Inh family metalloprotease inhibitor | OM, 0.992 | 0.437 | 0 | 177 |
| **2^{a}** | WP_00576 6217.1 | BbadC | OM, 1.000 | 0.643 | 0 | 551 |
| **3^{a}** | WP_00576 6273.1 | hypothetical protein A | OM, 0.992 | 0.698 | 1 | 124 |
| **4^{a}** | WP_00576 6494.1 | porin A | OM, 0.992 | 0.689 | 0 | 406 |
| **5^{a}** | WP_00576 6517.1 | TonB-dependent haemoglobin / transferrin / lactoferrin family receptor | OM, 1.000 | 0.464 | 0 | 731 |
| **6^{a}** | WP_03545 3117.1 | LPS-assembly protein LptD | OM, 1.000 | 0.735 | 1 | 791 |
| **7^{a}** | WP_00576 6622.1 | autotransporter outer membrane beta-barrel domain-containing protein A | OM, 0.983 | 0.652 | 0 | 800 |
| **8^{a}** | WP_00576 6636.1 | LysM peptidoglycan-binding domain-containing protein | OM, 0.886 | 0.453 | 0 | 397 |
| **9^{a}** | WP_00576 7307.1 | DUF1561 family protein | EC, 0.965 | 0.610 | 0 | 649 |
| **10^{a}** | WP_00576 7360.1 | BbadA | OM, 0.995 | 0.861 | 0 | 1,259 |
| **11^{a}** | WP_01180 7398.1 | flagellin | EC, 0.971 | 0.401 | 0 | 380 |
| **12^{a}** | WP_00576 7706.1 | autotransporter outer membrane beta-barrel domain-containing protein B | OM, 0.949 | 0.561 | 0 | 1,193 |
| **13^{a}** | WP_00576 7730.1 | flagellar hook protein FlgE | EC, 1.000 | 0.751 | 0 | 404 |
| **14^{a}** | WP_05100 7750.1 | hypothetical protein B | EC, 0.965 | 0.872 | 0 | 610 |
| **15^{a}** | WP_ 193741 240.1 | autotransporter outer membrane | EC, 0.964 | 0.652 | 0 | 297 |
| | | beta-barrel domain-containing protein C | | | | |
| **16^{a}** | WP_00576 7753.1 | autotransporter outer membrane beta-barrel domain-containing protein D | OM, 0.9₅2 | 0.855 | 0 | 825 |
| **17^{a}** | WP 00576 7762.1 | flagellar basal body L-ring protein FlgH | OM, 0.992 | 0.410 | 1 | 230 |
| **18^{a}** | WP_00576 7896.1 | porin B | OM, 1.000 | 0.424 | 0 | 284 |
| **19^{a}** | WP_00576 7899.1 | Pap31 | OM, 0.993 | 0.534 | 0 | 300 |
| **20a** | WP_00576 7902.1 | porin C | OM, 1.000 | 0.493 | 0 | 281 |
| **21^{b}** | WP_01180 7413.1 | autotransporter E | OM, 0.992 | 0.351 | 0 | 1,058 |
| **22^{c}** | WP_00576 6221.1 | BbadB | prediction failed | 0.670 | 1 | 1,235 |

### Example 3: Analysis of Peruvian patient sera

To evaluate *B. bacilliformis* IgG seroractivity, the inventors established an IFA protocol similar to those described earlier (20, 21). Infected cell cultures (HeLa-229 cells, *B. bacilliformis* KC583) were used as antigen and reactivity was tested technically using a rabbit-anti *B. bacilliformis* serum (reactive until 1:1,600) resulting in a bright specific fluorescence signal whereas rabbit pre-immune serum nor human blood donor serum showed no specific fluorescence signals (<1:80). Of 26 Peruvian patient sera, 12 sera were tested IgG-positive with a titer ≥1:320 (sera # 1, 2, 4, 6, 7, 8, 9, 16, 17, 19, 23, 24), 11 sera were tested equivocal (≥1:160 and <1:320: sera #14, 15, 18, 21, 26; titer 1:80: sera #3, 5, 10, 11, 13, 25) and 3 were tested IgG-negative (<1:80: sera #12, 20, 22; supplementary Figure 1A). Patient sera were further evaluated via immunoblotting using *B. bacilliformis* KC583 whole cell lysates. The most reactive patient sera (#1, 2, 4, 8, 9; supplementary Figure 1B) from both IFA and western blot, respectively, were pooled in equal amounts for later screening of the expression libraries and to analyze the immunoreactivity of the in silico predicted (Table 1) and recombinantly expressed antigens.

### Example 4: Characterization of the recombinant B. bacilliformis protein expression libraries

Random genomic expression libraries have been proven to represent valuable tools for the identification of immunogenic proteins (26). To complete the list from the in silico prediction and to minimize the risk of missing potential immunodominant targets, inducible genomic *B. bacilliformis* expression libraries were constructed and screened for immunodominant proteins by using (i) serum of an experimentally infected rabbit (KC583) and (ii) sera from *B. bacilliformis*-infected patients. For this purpose, bacterial genomic DNA fragments (1-4 kb) were cloned and expressed in three different reading frames. Transformation of the pre-cloning strain E. coli DH5α with the assembled plasmids (average insert size: 1,900 bp) resulted in ∼24,700 recombinant clones (pET28a: ∼9,100; pET28b: ∼5,400; pET28c: ∼10,200) covering more than 99% of the *B. bacilliformis* KC583 genome. A pooled plasmid preparation was done from each cloning approach creating the three final expression libraries in E. coli BL21 (DE3). For quality control, 32 randomly selected colonies of each reading frame variant were analyzed by colony PCR showing an insert frequency of >90% of different insert sizes (supplementary Figure 2). Control of protein production by anti-T7 tag immunoblotting revealed an expression rate of >96%. GroEL and flagellin were identified to be immunoreactive by screening ∼5.000 colonies using the experimental rabbit-anti *B. bacilliformis* serum demonstrating the functionality of the libraries (data not shown).

### Example 5: Systematic analysis of B. bacilliformis target proteins

To find immunoreactive antigens that were not predicted by the in silico Vaxign-tool, recombinant *B. bacilliformis* expression libraries were screened by using the pre-adsorbed pool of high titer patient sera (Figure 2). In total, 7,435 colonies were analyzed resulting in six colonies that proved consistently reactive after three rounds of screening and immunoblotting. Sequence analysis of the respective plasmid inserts revealed that all six colonies contained gene fragments coding for "autotransporter outer membrane beta-barrel domain-containing protein" (autotransporter E; protein accession: WP_011807413.1, Table 1).

In total, 20 antigen targets from the Vaxign prediction and, additionally, BbadB (a homologue of the immunodominant BadA from B. henselae) (27) were recombinantly expressed and immunoreactivity with sera of Peruvian patients was determined via immunoblotting. Proteins were analyzed for (i) protein production (technical control: anti-T7-tag antibodies), (ii) reactivity with rabbit serum (technical control) and (iii) reactivity with patient sera (using a pool of five high reactive patient sera). In total, seven proteins (LysM, BbadA, flagellin, FlgE, Pap31, BbadB) were reactive with the rabbit serum and 12 proteins (hypothetical protein A, porin A, TonB-dependent receptor, LysM, BbadA, hypothetical protein B, autotransporter C, FlgH, porin B, Pap31, BbadC, and BbadB) showed reactivity with Peruvian patient sera (Figure 3). For unclear reasons, recombinant expression of AprI/Inh family metalloprotease inhibitor was not possible, therefore, this protein was not included in further studies.

### Example 6: Serodiagnostic evaluation of immunoreactive B. bacilliformis proteins via line-blotting

Subsequently, 13 previously identified antigens (BbadC, porin A, TonB-dependent receptor, DUF1561, hypothetical protein B, FlgH, BbadA, Pap31, BbadB, hypothetical protein A, autotransporter C, porin B and autotransporter E) that were proven to be seroreactive (Figure 3) were printed on line blots (2.1 ng, 10.5 ng, 21 ng per antigen band, not all results shown; Figure 4 A-C). LysM was excluded due to its strong reactivity with human control sera in preliminary tests (data not shown).

Human sera were categorized in (i) IFA positive patient sera with an anti-*B*. *bacilliformis* IgG IFA titer ≥1:320; (n=12), (ii) equivocal or negative patient sera with a titer <1:320 (n=14) and for control, (iii) sera from German healthy blood donors without travel history to South America (n=96). None of the tested sera showed a specific signal for BbadC, porin A, TonB-dependent receptor, FlgH, hypothetical protein A and autotransporter C whereas DUF1561 was highly cross-reactive (n=11/96; 11,5%) with control sera (data not shown). BbadA and BbadB both reacted each with n=1 (8.3%) of the IFA positive patient sera but were also highly reactive with the control sera (n=10; 9.6% and n=6; 6.3%, respectively). Pap31, previously considered as a potential antigenic candidate (9, 28) showed nearly no reactivity with the IFA-positive (n=1 8.3%) and one IFA-equivocal patient serum (n=1; 7.1%) revealing no statistically significant difference to control sera (Figure 4D). Hypothetical protein B reacted with n=2 (16.6%) of the IFA-positive sera. Porin B reacted with n=3 (25%) of the IFA-positive sera and statistical analysis revealed a significant difference between IFA positive patient and control sera. Autotransporter E displayed the strongest immunoreactivity among the tested antigens. Here, n=7 (58.3%) of the IFA positive patient sera were reactive whereas none of the controls showed a positive signal leading to a statistically significant difference. Interestingly, two out of all 12 IFA-positive sera (patient sera #16 and #17) were non-reactive with any antigen.

### Example 7: Serodiagnostic evaluation of immunoreactive B. bacilliformis proteins via ELISA

The best reacting antigens of the line blots (porin B, autotransporter E and hypothetical protein B; see above) were further tested in an ELISA format to detect IgG-antibodies. Two different antigen combinations [(i) porin B, autotransporter E, (ii) porin B, autotransporter E, hypothetical protein B] were tested for IgG antibodies either with Peruvian patient sera with an IFA titer >1:320 (n=12), sera with a titer of <1:320 (n=14) and control sera (n=96). Both ELISAs allowed a clear detection from IFA-positive sera (Figure 5). A receiver operating characteristic curve (ROC) analysis for the ELISA using a combination of porin B and autotransporter E revealed an area under the ROC curve (AUC) of 0.9335. Adjusting the cut-off at an optical density (OD450) of 0.38 resulted in a sensitivity and specificity each of 100% for IFA-positive sera and 69.2% sensitivity and 100% specificity for all Peruvian patient sera, and adjusting the cut-off at 0.29 resulted in 100% sensitivity and 94.8% specificity for IFA-positive sera and 80.8% sensitivity and 94.8% specificity for all Peruvian patient sera, respectively. For the ELISA using a combination of porin B, autotransporter E and hypothetical protein B the AUC score was 0.9083 and the cut-off was set to an optical density (OD450) of 0.34 revealing 100% sensitivity and 93.8% specificity for IFA-positive and 76.6% sensitivity and 93.8% specificity for all Peruvian patient sera, respectively. These data suggest that the combination of porin B and autotransporter E is sufficient to detect *B. bacilliformis* antibodies via ELISA.

### REFERENCES

The references are:
1. Gomes C, Pons MJ, del Valle Mendoza J, Ruiz J. 2016. Carrion's disease: An eradicable illness? Infect Dis Poverty.
2. Battisti JM, Lawyer PG, Minnick MF. 2015. Colonization of Lutzomyia verrucarum and Lutzomyia longipalpis Sand Flies (Diptera: Psychodidae) by Bartonella bacilliformis, the Etiologic Agent of Carrión's Disease. PLoS Negl Trop Dis.
3. Lydy SL, Eremeeva ME, Asnis D, Paddock CD, Nicholson WL, Silverman DJ, Dasch GA. 2008. Isolation and characterization of Bartonella bacilliformis from an expatriate Ecuadorian. J Clin Microbiol.
4. Garcia-Quintanilla M, Dichter AA, Guerra H, Kempf VAJ. 2019. Carrion's disease: More than a neglected disease. Parasites and Vectors 12:1-12.
5. Pons MJ, Gomes C, Aguilar R, Barrios D, Aguilar-Luis MA, Ruiz J, *Dobano C, del Valle-Mendoza J, Moncunill G. 2017. Immunosuppressive and angiogenic cytokine profile associated with Bartonella bacilliformis infection in post-outbreak and endemic areas of Carrion's disease in Peru. PLoS Negl Trop Dis 11:1-16.
6. Noguchi H, Muller HR, Tilden EB, Tyler JR. 1929. Etiology of oroya fever: XV. Effect of immune serum on the course of bartonella bacilliformis infection in macacus rhesus. J Exp Med.
7. Ellis BA, Rotz LD, Leake JAD, Samalvides F, Bernable J, Ventura G, Padilla C, Villaseca P, Beati L, Regnery R, Childs JE, Olson JG, Carrillo CP. 1999. An outbreak of acute Bartonellosis (Oroya fever) in the Urubamba region of Peru, 1998. Am J Trop Med Hyg.
8. Minnick MF, Anderson BE, Lima A, Battisti JM, Lawyer PG, Birtles RJ. 2014. Oroya fever and verruga peruana: Bartonelloses unique to South America. PLoS Negl Trop Dis 8:62919.
9. Gomes C, Palma N, Pons MJ, Magallón-Tejada A, Sandoval I, Tinco-Valdez C, Gutarra C, del Valle-Mendoza J, Ruiz J, Matsuoka M. 2016. Succinyl-CoA Synthetase: New Antigen Candidate of Bartonella bacilliformis. PLoS Negl Trop Dis.
10. Knobloch J, Solano L, Alvarez O, Delgado E. 1985. Antibodies to Bartonella bacilliformis as determined by fluorescence antibody test, indirect haemagglutination and ELISA. Trop Med Parasitol.
11. Angkasekwinai N, Atkins EH, Romero S, Grieco J, Chao CC, Ching WM. 2014. An evaluation study of Enzyme-Linked Immunosorbent Assay (ELISA) using recombinant protein Pap31 for detection of antibody against Bartonella bacilliformis infection among the Peruvian population. Am J Trop Med Hyg.
12. Henriquez-Camacho C, Ventosilla P, Minnick MF, Ruiz J, Maguiña C. 2015. Proteins of bartonella bacilliformis: Candidates for vaccine development. Int J Pept 2015.
13. Riess T, Dietrich F, Schmidt K V., Kaiser PO, Schwarz H, Schäfer A, Kempf VAJ. 2008. Analysis of a novel insect cell culture medium-based growth medium for Bartonella species. Appl Environ Microbiol.
14. Studier FW. 2005. Protein production by auto-induction in high density shaking cultures. Protein Expr Purif.
15. Wick RR, Judd LM, Gorrie CL, Holt KE. 2017. Unicycler: Resolving bacterial genome assemblies from short and long sequencing reads. PLoS Comput Biol 13:1-22.
16. Li H. 2018. Minimap2: Pairwise alignment for nucleotide sequences. Bioinformatics.
17. Darling ACE, Mau B, Blattner FR, Perna NT. 2004. Mauve: Multiple alignment of conserved genomic sequence with rearrangements. Genome Res.
18. Seemann T. 2014. Prokka: Rapid prokaryotic genome annotation. Bioinformatics 30:2068-2069.
19. He Y, Xiang Z, Mobley HLT. 2010. Vaxign: The first web-based vaccine design program for reverse vaccinology and applications for vaccine development. J Biomed Biotechnol 2010.
20. Regnery RL, Olson JG, Perkins BA, Bibb W. 1992. Serological response to "Rochalimaea henselae" antigen in suspected cat-scratch disease. Lancet.
21. Chamberlin J, Laughlin L, Gordon S, Romero S, Solórzano N, Regnery RL. 2000. Serodiagnosis of Bartonella bacilliformis infection by indirect fluorescence antibody assay: Test development and application to a population in an area of bartonellosis endemicity. J Clin Microbiol.
22. Hang L, John M, Asaduzzaman M, Bridges EA, Vanderspurt C, Kirn TJ, Taylor RK, Hillman JD, Progulske-Fox A, Handfield M, Ryan ET, Calderwood SB. 2003. Use of in vivo-induced antigen technology (IVIAT) to identify genes uniquely expressed during human infection with Vibrio cholerae. Proc Natl Acad Sci U S A.
23. John M, Kudva IT, Griffin RW, Dodson AW, McManus B, Krastins B, Sarracino D, Progulske-Fox A, Hillman JD, Handfield M, Tarr PI, Calderwood SB. 2005. Use of in vivo-induced antigen technology for identification of Escherichia coli O157:H7 proteins expressed during human infection. Infect Immun.
24. Pizza M, Scarlato V, Masignani V, Giuliani MM, Aricò B, Comanducci M, Jennings GT, Baldi L, Bartolini E, Capecchi B, Galeotti CL, Luzzi E, Manetti R, Marchetti E, Mora M, Nuti S, Ratti G, Santini L, Savino S, Scarselli M, Storni E, Zuo P, Broeker M, Hundt E, Knapp B, Blair E, Mason T, Tettelin H, Hood DW, Jeffries AC, Saunders NJ, Granoff DM, Venter JC, Moxon ER, Grandi G, Rappuoli R. 2000. Identification of vaccine candidates against serogroup B meningococcus by whole-genome sequencing. Science (80- ).
25. Maione D, Margarit I, Rinaudo CD, Masignani V, Mora M, Scarselli M, Tettelin H, Brettoni C, Iacobini ET, Rosini R, D'Agostino N, Miorin L, Buccato S, Mariani M, Galli G, Nogarotto R, Dei VN, Vegni F, Fraser C, Mancuso G, Teti G, Madoff LC, Paoletti LC, Rappuoli R, Kasper DL, Telford JL, Grandi G. 2005. Immunology: Identification of a universal group B Streptococcus vaccine by multiple genome screen. Science (80- ).
26. Miller L, Richter M, Hapke C, Stern D, Nitsche A. 2011. Genomic expression libraries for the identification of cross-reactive orthopoxvirus antigens. PLoS One 6:1-10.
27. Wagner CL, Riess T, Linke D, Eberhardt C, Schäfer A, Reutter S, Maggi RG, Kempf VAJ. 2008. Use of Bartonella adhesin A (BadA) immunoblotting in the serodiagnosis of Bartonella henselae infections. Int J Med Microbiol.
28. Ruiz J, Gomes C. 2020. In silico analysis of Pap31 from Bartonella bacilliformis and other Bartonella spp. Infect Genet Evol.
29. Clemente NS, Ugarte-Gil C, Solorzano N, Maguiña C, Moore D. 2016. An outbreak of bartonella bacilliformis in an Endemic Andean community. PLoS One.
30. Rappuoli R. 2000. Reverse vaccinology. Curr Opin Microbiol.
31. Qamar MTU, Shokat Z, Muneer I, Ashfaq UA, Javed H, Anwar F, Bari A, Zahid B, Saari N. 2020. Multiepitope-based subunit vaccine design and evaluation against respiratory syncytial virus using reverse vaccinology approach. Vaccines.
32. Dichter AA, Schultze TG, Becker SA, Tsukayama P, Kempf VAJ. 2020. Complete Genome Sequence of Bartonella bacilliformis Strain KC584 (ATCC 35686). Microbiol Resour Announc.
33. Jwang B, Dewing P, Fikrig E, Flavell RA. 1995. The hook protein of Borrelia burgdorferi, encoded by the flgE gene, is serologically recognized in Lyme disease. Clin Diagn Lab Immunol.
34. Fikrig E, Huguenel ED, Berland R, Rahn DW, Hardin JA, Flavell RA. 1992. Serologic Diagnosis of Lyme Disease using Recombinant Outer Surface Proteins A and B and Flagellin. J Infect Dis.
35. Wells TJ, Tree JJ, Ulett GC, Schembri MA. 2007. Autotransporter proteins: Novel targets at the bacterial cell surface. FEMS Microbiol Lett.
36. Henderson IR, Navarro-Garcia F, Desvaux M, Fernandez RC, Ala'Aldeen D. 2004. Type V Protein Secretion Pathway: the Autotransporter Story. Microbiol Mol Biol Rev.
37. Thibau A, Dichter AA, Vaca DJ, Linke D, Goldman A, Kempf VAJ. 2020. Immunogenicity of trimeric autotransporter adhesins and their potential as vaccine targets. Med Microbiol Immunol.
38. Secundino I, López-Macías C, Cervantes-Barragán L, Gil-Cruz C, Ríos-Sarabia N, Pastelin-Palacios R, Villasis-Keever MA, Becker I, Puente JL, Calva E, Isibasi A. 2006. Salmonella porins induce a sustained, lifelong specific bactericidal antibody memory response. Immunology.
39. Dalsass M, Brozzi A, Medini D, Rappuoli R. 2019. Comparison of open-source reverse vaccinology programs for bacterial vaccine antigen discovery. Front Immunol.
40. Knobloch J, Schreiber M. 1990. Bb65, a major immunoreactive protein of Bartonella bacilliformis. Am J Trop Med Hyg.
41. Anderson BE, Neuman MA. 1997. Bartonella spp. as emerging human pathogens. Clin Microbiol Rev.
42. Minnick MF, Smitherman LS, Samuels DS. 2003. Mitogenic Effect of Bartonella bacilliformis on Human Vascular Endothelial Cells and Involvement of GroEL. Infect Immun 71:6933-6942.
43. Padmalayam I, Kelly T, Baumstark B, Massung R. 2000. Molecular cloning, sequencing, expression, and characterization of an immunogenic 43-kilodalton lipoprotein of Bartonella bacilliformis that has homology to NlpD/LppB. Infect Immun.
44. Knobloch J. 1988. Analysis and preparation of Bartonella bacilliformis antigens. Am J Trop Med Hyg.

## Claims

1. **A method for inducing an immune response against *Bartonella bacilliformis* in a subject,** the method comprising the steps of
(i) Providing an immunological composition comprising at least one isolated immunodominant protein selected from a protein shown in any one of SEQ ID NO: 1-14, or comprising an isolated protein fragment having at least 8 consecutive amino acids of an amino acid sequence shown in any of SEQ ID NO: 1-14, or providing a nucleic acid sequence encoding the immunodominant protein or protein fragment thereof;
(ii) Administering the immunological composition to the subject in an amount sufficient to induce an immune response.

2. The method of claim 1, wherein the immunological composition does not comprise *Bartonella bacilliformis* or any fragments thereof not being an immunodominant protein or fragment thereof.

3. The method of claim 1, wherein the method is a non-therapeutic method for the generation of antibodies specific for the immunodominant protein or fragment thereof.

4. The method of claim 3, comprising the further step of isolated antibody producing cells from the subject.

5. **A method for producing a vaccine composition for vaccinating a subject against *Bartonella bacilliformis,* the method comprising the steps of**
(i) Providing at least one isolated immunodominant protein selected from a protein shown in any one of SEQ ID NO: 1-14, or comprising an isolated protein fragment having at least 8 consecutive amino acids of an amino acid sequence shown in any of SEQ ID NO: 1-14; or providing a nucleic acid sequence encoding the immunodominant protein or protein fragment thereof,
(ii) Formulating the immunodominant protein or fragment thereof of (i) with at least one carrier and/or excipient suitable for use in a vaccine composition, such as a pharmaceutically acceptable carrier and/or excipient.

6. **A method of detecting a *Bartonella bacilliformis*-infected subject,** said method comprising detecting in a biological sample from the subject at least one protein selected from a group comprising SEQ ID NO: 1-14 and conservative variants thereof, or antibodies against at least one protein selected from a group comprising SEQ ID NO: 1-14 and conservative variants thereof in an immunoassay, wherein detection of said protein or said antibodies indicates that the subject is infected and not vaccinated.

7. The method of claim 6, said method comprising:
(i) contacting a biological sample with immobilized primary antibodies on the solid support, wherein the primary antibodies are specific to SEQ ID NO: 1-14 and conservative variants thereof and the primary antibodies form an antibody-antigen complex with SEQ ID NO: 1-14 and conservative variants thereof; and
(ii) detecting the presence of antigens bound to the primary antibodies using conjugated secondary antibodies raised against a protein selected from a group comprising SEQ ID NO: 1-14 and conservative variants thereof, wherein the detection of said antigens indicates that the subject is infected and not vaccinated with *Bartonella bacilliformis.*

8. The method of claim 6, said method comprising:
(i) contacting a biological sample with the immobilized antigenic immunodominant protein selected from a group comprising SEQ ID NO: 1-14 and conservative variants thereof on the solid support, wherein the immobilized antigenic immunodominant protein form an antigen- antibody complex with antibodies specific to said antigenic proteins; and
(ii) detecting the presence of antibodies bound to the antigenic immunodominant protein using conjugated secondary antibodies, wherein the detection of said antibodies indicates that the animal is infected and not vaccinated with *Bartonella bacilliformis.*

9. **An antibody or antibody-serum,** produced with a method according to claim 3 or 4.

10. **A lateral flow immunoassay device for detecting** ***Bartonella bacilliformis** -* **infected subjects,** comprising:
(i) a sample pad configured for receiving a biological sample;
(ii) a conjugate pad comprising conjugated antibodies raised against a protein selected from a group comprising SEQ ID NO: 1-14 and conservative variants thereof;
(iii) a lateral flow membrane comprising a test line and a control line, wherein the test line comprises antibodies raised against a protein selected from a group comprising SEQ ID NO: 1-14 and conservative variants thereof, and the control line; and
(iv) an absorbent pad.

11. **A lateral flow immunoassay device for detecting** ***Bartonella bacilliformis** -* **infected subjects,** comprising:
(i) a sample pad configured for receiving a biological sample;
(ii) a conjugate pad comprising conjugated antibody;
(iii) a lateral flow membrane comprising a test line and a control line, wherein the test line comprises antigens comprising the amino acid sequence selected from a group comprising SEQ ID NO: 1-14 and conservative variants thereof, and the control line comprises unrelated proteins and lipo-polysaccharides; and
(iv) an absorbent pad.

12. **Use of a protein selected from a group comprising SEQ ID NO: 1-14, or conservative variants and combinations thereof for detecting *Bartonella bacilliformis* in a subject.**

13. **An immunoassay kit for detecting *Bartonella bacilliformis* in a subject,** comprising a solid support immobilized with primary antibodies raised against a protein selected from a group comprising SEQ ID NO: 1-14 and conservative variants thereof.

14. **An immunoassay kit for detecting *Bartonella bacilliformis, preferably* antibodies against *Bartonella bacilliformis,* in a subject,** comprising a solid support immobilized with a protein selected from a group comprising SEQ ID NO: 1-14 and conservative variants thereof.

15. **A vaccine composition,** comprising
(i) at least one isolated immunodominant protein selected from a protein shown in any one of SEQ ID NO: 1-14, or comprising an isolated protein fragment having at least 8 consecutive amino acids of an amino acid sequence shown in any one of SEQ ID NO: 1-14; and/or
(ii) an expressible nucleic acid comprising a nucleic acid sequence coding for at least one isolated immunodominant protein selected from a protein shown in any one of SEQ ID NO: SEQ ID NO: 1-14, or coding for an isolated protein fragment having at least 8 consecutive amino acids of an amino acid sequence shown in any one of SEQ ID NO: 1-14;
together with a pharmaceutically acceptable carrier and/or excipient.
